# EUROPÄISCHE PATENTSCHRIFT

(11) **EP 2 401 254 B1**
(45) Veröffentlichungstag und Bekanntmachung des Hinweises auf die Patenterteilung: **19.06.2013**
(21) Anmeldenummer: 10706602.9
(22) Anmeldetag: 25.02.2010
(51) Int. Cl.: C07D 209/34, C07D 403/10, C07D 403/14

(54) **CHINONVERBINDUNGEN ALS DOTIERSTOFF IN DER ORGANISCHEN ELEKTRONIK**
QUINONE COMPOUNDS AS DOPANTS IN ORGANIC ELECTRONICS
COMPOSÉS QUINONIQUES CONVENANT COMME IMPURETÉ DE DOPAGE EN ÉLECTRONIQUE ORGANIQUE

(30) Priorität: 26.02.2009 EP 09153776
(43) Veröffentlichungstag der Anmeldung: 04.01.2012
(73) Patentinhaber: Novaled AG, 01307 Dresden (DE)
(72) Erfinder: QU, Jianqiang, 67061 Ludwigshafen (DE); LANGER, Nicolle, 64646 Heppenheim (DE); BRUDER, Ingmar, 67059 Ludwigshafen (DE); MÜLLER, Imke Britta, 69118 Heidelberg (DE); WATANABE, Soichi, 68161 Mannheim (DE)
(74) Vertreter: Bittner, Thomas L.
(86) Internationale Anmeldenummer: PCT/EP2010/052400
(87) Internationale Veröffentlichungsnummer: WO 2010/097433

(56) Entgegenhaltungen:
- EP-A1- 1 596 445

## Beschreibung

Die vorliegende Erfindung betrifft neue Chinonverbindungen und deren Verwendung als Dotierstoff in der organischen Elektronik.

Es wird erwartet, dass zukünftig in vielen Bereichen der Elektronikindustrie neben den klassischen anorganischen Halbleitern zunehmend auch organische Halbleiter auf Basis von niedermolekularen oder polymeren Materialien eingesetzt werden. Diese weisen vielfach Vorteile gegenüber den klassischen anorganischen Halbleitern auf, beispielsweise eine bessere Substratkompatibilität und eine bessere Verarbeitbarkeit der auf ihnen basierenden Halbleiterbauteile. Sie erlauben die Verarbeitung auf flexiblen Substraten und ermöglichen es, ihre Grenzorbitalenergien mit den Methoden des Molecular Modellings auf den jeweiligen Anwendungsbereich genau anzupassen. Die "Organische Elektronik" beschäftigt sich schwerpunktmäßig mit der Entwicklung neuer Materialien und Fertigungsprozesse für die Herstellung elektronischer Bauelemente auf der Basis organischer Halbleiterschichten. Dazu zählen vor allem organische Leuchtdioden (organische lichtemittierende Dioden, organic light emitting diods, OLED), organische Feldeffekttransistoren (Organic Field-Effect Transistors, OFET) sowie die organische Photovoltaik. Organischen Feldeffekttransistoren wird ein großes Entwicklungspotential, beispielsweise in Speicherelementen und integrierten optoelektronischen Vorrichtungen, zugeschrieben. In organischen lichtemittierenden Dioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigeren Stromverbrauchs eignen sich Vorrichtungen, die OLEDs enthalten, insbesondere für mobile Anwendungen, zum Beispiel für Anwendungen in Mobiltelefonen, Laptops usw. Ein großes Entwicklungspotential wird auch Materialien zugeschrieben, die möglichst große Transportweiten und hohe Mobilitäten für lichtinduzierte angeregte Zustände (hohe Excitonendiffusionslängen) besitzen und die sich somit vorteilhaft für einen Einsatz als aktives Material in organischen Solarzellen, speziell so genannten excitonischen Solarzellen, eignen.

Es ist bekannt, die elektronischen Eigenschaften von Siliziumhalbleitern durch Dotierung zu modifizieren. Dabei wird durch Erzeugung von Ladungsträgern eine Erhöhung der Leitfähigkeit sowie je nach Art des verwendeten Dotierstoffs (Dotanden) eine Modifizierung des Fermi-Niveaus des Halbleiters erreicht.

Es ist weiterhin bekannt, dass sich auch organische Halbleiter durch Dotierung hinsichtlich ihrer elektrischen Leitfähigkeit beeinflussen lassen. Organische halbleitende Materialien können aus Verbindungen mit guten Elektronendonator-Eigenschaften oder guten Elektronenakzeptor-Eigenschaften aufgebaut werden. Zum Dotieren von Elektronendonator-Materialien sind starke Elektronen-Akzeptoren wie Tetracyanochinondimethan (TCNQ) oder 2,3,5,6-Tetrafluorotetracyano-1,4-benzochinondimethan (F4TCNQ) bekannt. Diese erzeugen durch Elektronentransferprozesse in elektronendonatorartigen Basismaterialien (Löchertransportmaterialien) so genannte Löcher, durch deren Anzahl und Beweglichkeit sich die Leitfähigkeit des Basismaterials verändert. Als Materialien mit Löchertransporteigenschaften sind beispielsweise N,N'-perarylierte Benzidine, N,N',N"-perarylierte Starburstverbindungen oder bestimmte Metallphthalocyanine, wie insbesondere Zinkphthalocyanin (ZnPc), bekannt.

Die EP 1 596 445 A1 beschreibt die Verwendung von Chinonderivaten, die unter gleichen Verdampfungsbedingungen eine geringere Flüchtigkeit als Tetrafluortetracyanochinodimethan (F₄TCNQ) aufweisen, zur Dotierung eines organischen Halbleitermatrixmaterials. Konkret werden mit Fluor und/oder Chlor substituierte 1,4-Chinondiimine sowie 1,4,5,8-Tetrahydro-1,4,5,8-tetrathia-2,3,6,7-tetracyanoanthrachinon (CN₄TTAQ) eingesetzt. Nachteilig speziell an halogenhaltigen Dotierstoffen ist ihre Neigung zur Migration in benachbarte undotierte Schichten. Zudem ist auch die Lebensdauer daraus hergestellter elektronischer Bauelemente und insbesondere von OLEDs noch verbesserungswürdig.

Es besteht weiterhin ein großer Bedarf an neuen Dotierstoffen für die organische Elektronik mit vorteilhaften elektronischen und anwendungstechnischen Eigenschaften.

Überraschenderweise wurde nun gefunden, dass Chinonderivate, die eine Kombination von Cyanogruppen und Phthalimidgruppen aufweisen, sich durch ihre leichte Zugänglichkeit und sehr gute Eigenschaften für einen Einsatz als Dotierstoff in der organischen Elektronik auszeichnen. Dabei handelt es sich speziell auch um halogenfreie Dotierstoffe. Sie zeichnen sich insbesondere durch eine hohe Thermostabilität aus und/oder lassen sich bei hohen Temperaturen sublimieren. Vorzugsweise kann bei einem Einsatz in elektronischen Bauteilen gegenüber bekannten Dotierstoffen eine unerwünschte Migration in benachbarte undotierte Schichten vermieden bzw. stark vermindert werden. Elektronische Bauteile auf Basis der erfindungsgemäßen Chinonderivate zeichnen sich gegenüber bekannten Dotierstoffen in der Regel durch eine deutlich längere Lebensdauer aus.

Ein erster Gegenstand der Erfindung ist ein Chinonderivat der allgemeinen Formel (I) 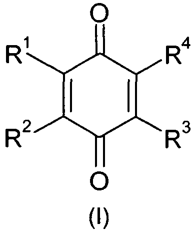 worin
1, 2 oder 3 der Reste R¹ bis R⁴ für CN stehen,
1, 2 oder 3 der Reste R¹ bis R⁴ unabhängig voneinander für einen Phthalimidrest der allgemeinen Formel (II) 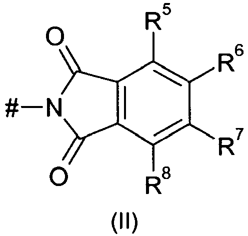 stehen, worin
# für die Verknüpfungsstelle zu einem Ringkohlenstoffatom des Chinonrings steht,
R⁵, R⁶, R⁷ und R⁸ unabhängig voneinander für Wasserstoff, Fluor, Chlor, Brom, Cyano, Nitro, Nitroso, Formyl, Acyl, COOH, Carboxylat, Alkylcarbonyloxy, Carbamoyl, SO₃H, Sulfonat, Sulfamino, Sulfamid, Amidino, NE¹E², oder für unsubstituiertes oder substituiertes Alkyl, Alkoxy, Alkylamino, Alkylthio, Alkylsulfinyl, Alkylsulfonyl, Alkenyl, Alkadienyl, Alkinyl, Cycloalkyl, Bicycloalkyl, Cycloalkenyl, Heterocycloalkyl, Aryl oder Heteroaryl stehen, wobei E¹ und E² unabhängig voneinander für Wasserstoff, Alkyl, Cycloalkyl oder Aryl stehen,
wobei jeweils zwei benachbarte Reste R⁵ bis R⁸ gemeinsam mit den Kohlenstoffatomen des Benzolkerns, an die sie gebunden sind, auch für ein kondensiertes Ringsystem mit 1, 2 oder 3 weiteren Ringen stehen können, und
die Reste R¹ bis R⁴, soweit vorhanden, die nicht für CN oder für einen Phthalimidrest der allgemeinen Formel (II) stehen, unabhängig voneinander ausgewählt sind unter Wasserstoff, Fluor, Chlor, Brom, Cyano, Nitro, Nitroso, Formyl, Acyl, COOH, Carboxylat, Alkylcarbonyloxy, Carbamoyl, SO₃H, Sulfonat, Sulfamino, Sulfamid, Amidino, NE³E⁴, oder unsubstituiertem oder substituiertem Alkyl, Alkoxy, Alkylamino, Alkylthio, Alkylsulfinyl, Alkylsulfonyl, Alkenyl, Alkadienyl, Alkinyl, Cycloalkyl, Bicycloalkyl, Cycloalkenyl, Heterocycloalkyl, Aryl oder Heteroaryl, wobei E³ und E⁴ unabhängig voneinander für Wasserstoff, Alkyl, Cycloalkyl oder Aryl stehen,
wobei R¹ und R² und/oder R³ und R⁴ gemeinsam mit den Kohlenstoffatomen des Chinonrings, an die sie gebunden sind, auch für ein kondensiertes Ringsystem mit 1, 2 oder 3 weiteren Ringen stehen können.

Im Rahmen der vorliegenden Erfindung umfasst der Ausdruck "Alkyl" geradkettiges oder verzweigtes Alkyl. Vorzugsweise handelt es sich um geradkettiges oder verzweigtes C₁-C₃₀-Alkyl, insbesondere um C₁-C₂₀-Alkyl und ganz besonders bevorzugt C₁-C₁₂-Alkyl. Beispiele für Alkylgruppen sind insbesondere Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Hexadecyl, n-Octadecyl und n-Eicosyl.

Der Ausdruck Alkyl umfasst auch Alkylreste, deren Kohlenstoffketten durch eine oder mehrere nicht benachbarte Gruppen, die ausgewählt sind unter -O-, -S-, -NR^{e}-, -C(=O)-, -S(=O)- und/oder -S(=O)₂- unterbrochen sein kann. R^{e} steht vorzugsweise für Wasserstoff, Alkyl, Cycloalkyl, Heterocycloalkyl, Aryl oder Hetaryl. Der Ausdruck Alkyl umfasst auch substituierte Alkylreste. Substituierte Alkylgruppen können in Abhängigkeit von der Länge der Alkylkette einen oder mehrere (z. B. 1, 2, 3, 4, 5 oder mehr als 5) Substituenten aufweisen. Diese sind vorzugsweise unabhängig voneinander ausgewählt unter Cycloalkyl, Heterocycloalkyl, Aryl, Hetaryl, Halogen, Hydroxy, Mercapto, COOH, Carboxylat, SO₃H, Sulfonat, NE¹E², Nitro und Cyano, wobei E¹ und E² unabhängig voneinander für Wasserstoff, Alkyl, Cycloalkyl, Heterocycloalkyl, Aryl oder Hetaryl stehen. Halogensubstituenten sind vorzugsweise Fluor, Chlor oder Brom.

Carboxylat und Sulfonat stehen für ein Derivat einer Carbonsäurefunktion bzw. einer Sulfonsäurefunktion, insbesondere für ein Metallcarboxylat oder -sulfonat, eine Carbonsäureester- oder Sulfonsäureesterfunktion oder eine Carbonsäure- oder Sulfonsäureamidfunktion. Cycloalkyl-, Heterocycloalkyl-, Aryl- und Hetarylsubstituenten der Alkylgruppen können ihrerseits unsubstituiert oder substituiert sein; geeignete Substituenten sind die nachfolgend für diese Gruppen genannten.

Die vorstehenden Ausführungen zu Alkyl gelten auch für die Alkylteile in Alkoxy, Alkylamino, Alkylthio, Alkylsulfinyl, Alkylsulfonyl, Alkylcarbonyloxy, etc.

Durch Aryl substituierte Alkylreste ("Arylalkyl") weisen wenigstens eine, wie nachfolgend definierte, unsubstituierte oder substituierte Arylgruppe auf. Dabei kann die Alkylgruppe in "Arylalkyl" wenigstens einen weiteren Substituenten tragen und/oder durch eine oder mehrere nicht benachbarte Gruppen, die ausgewählt sind unter -O-, -S-, -NR^{e}-, -CO- und/oder -SO₂- unterbrochen sein. Arylalkyl steht vorzugsweise für Phenyl-C₁-C₁₀-alkyl, besonders bevorzugt für Phenyl-C₁-C₄-alkyl, z. B. für Benzyl, 1-Phenethyl, 2-Phenethyl, 1-Phenprop-1-yl, 2-Phenprop-1-yl, 3-Phenprop-1-yl, 1-Phenbut-1-yl, 2-Phenbut-1-yl, 3-Phenbut-1-yl, 4-Phenbut-1-yl, 1-Phenbut-2-yl, 2-Phenbut-2-yl, 3-Phenbut-2-yl, 4-Phenbut-2-yl, 1-(Phenmeth)-eth-1-yl, 1-(Phenmethyl)-1-(methyl)-eth-1-yl oder -(Phenmethyl)-1-(methyl)-prop-1-yl; vorzugsweise für Benzyl und 2-Phenethyl.

Der Ausdruck "Alkenyl" umfasst im Sinne der vorliegenden Erfindung geradkettige und verzweigte Alkenylgruppen, die in Abhängigkeit von der Kettenlänge eine oder mehrere, nicht kumulierte Kohlenstoff-Kohlenstoff-Doppelbindungen (z. B. 1, 2, 3, 4 oder mehr als 4) tragen können. Alkenyl, das zwei Doppelbindungen aufweist, wird im Folgenden auch als Alkadienyl bezeichnet. Bevorzugt sind C₂-C₁₈-, besonders bevorzugt C₂-C₁₂-Alkenylgruppen. Der Ausdruck "Alkenyl" umfasst auch substituierte Alkenylgruppen, welche einen oder mehrere (z. B. 1, 2, 3, 4 5 oder mehr als 5) Substituenten tragen können. Geeignete Substituenten sind z. B. ausgewählt unter Cycloalkyl, Heterocycloalkyl, Aryl, Hetaryl, Halogen, Hydroxy, Mercapto, COOH, Carboxylat, SO₃H, Sulfonat, NE³E⁴, Nitro und Cyano, wobei E³ und E⁴ unabhängig voneinander für Wasserstoff, Alkyl, Cycloalkyl, Heterocycloalkyl, Aryl oder Hetaryl stehen.

Alkenyl steht dann beispielsweise für Ethenyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl und dergleichen. Die Ausführungen zu Alkenyl gelten auch für die Alkenylgruppen in Alkenyloxy, Alkenylthio, etc.

Der Ausdruck "Alkinyl" umfasst unsubstituierte oder substituierte Alkinylgruppen, die eine oder mehrere nicht benachbarte Dreifachbindungen aufweisen, wie Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl, 5-Hexinyl, und dergleichen. Die Ausführungen zu Alkinyl gelten auch für die Alkinylgruppen in Alkinyloxy, Alkinylthio, etc. Substituierte Alkinyle tragen vorzugsweise einen oder mehrere (z. B. 1, 2, 3, 4, 5 oder mehr als 5) der zuvor für Alkyl genannten Substituenten.

Der Ausdruck "Cycloalkyl" umfasst im Rahmen der vorliegenden Erfindung unsubstituierte als auch substituierte Cycloalkylgruppen, vorzugsweise C₃-C₈-Cycloalkylgruppen wie Cyclopropyl, Cyclobutyl, Cyclopentyl, Cyclohexyl, Cycloheptyl oder Cyclooctyl, insbesondere C₅-C₈-Cycloalkyl. Substituierte Cycloalkylgruppen können einen oder mehrere (z. B. 1, 2, 3, 4, 5 oder mehr als 5) Substituenten aufweisen. Diese sind vorzugsweise unabhängig voneinander ausgewählt unter Alkyl sowie den zuvor für die Alkylgruppen genannten Substituenten. Die Cycloalkylgruppen tragen im Falle einer Substitution vorzugsweise eine oder mehrere, beispielsweise eine, zwei, drei, vier oder fünf C₁-C₆-Alkylgruppen.

Beispiele für bevorzugte Cycloalkylgruppen sind Cyclopentyl, 2- und 3-Methylcyclopentyl, 2- und 3-Ethylcyclopentyl, Cyclohexyl, 2-, 3- und 4-Methylcyclohexyl, 2-, 3-und 4-Ethylcyclohexyl, 2-, 3- und 4-Propylcyclohexyl, 2-, 3- und 4-Isopropylcyclohexyl, 2-, 3- und 4-Butylcyclohexyl, 2-, 3- und 4-sec.-Butylcyclohexyl, 2-, 3- und 4-tert.-Butylcyclohexyl, Cycloheptyl, 2-, 3- und 4-Methylcycloheptyl, 2-, 3- und 4-Ethylcycloheptyl, 2-, 3- und 4-Propylcycloheptyl, 2-, 3- und 4-Isopropylcycloheptyl, 2-, 3- und 4-Butylcycloheptyl, 2-, 3- und 4-sec.-Butylcycloheptyl, 2-, 3- und 4-tert.-Butylcycloheptyl, Cyclooctyl, 2-, 3-, 4- und 5-Methylcyclooctyl, 2-, 3-, 4- und 5-Ethylcyclooctyl, 2-, 3-, 4- und 5-Propylcyclooctyl.

Der Ausdruck Cycloalkenyl umfasst unsubstituierte und substituierte einfach ungesättigte Kohlenwasserstoffgruppen mit 3 bis 8, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl und dergleichen. Geeignete Substituenten sind die zuvor für Cycloalkyl genannten.

Der Ausdruck Bicycloalkyl umfasst vorzugsweise bicyclische Kohlenwasserstoffreste mit 5 bis 10 C-Atomen wie Bicyclo[2.2.1]hept-1-yl, Bicyclo[2.2.1]hept-2-yl, Bicyclo[2.2.1]hept-7-yl, Bicyclo[2.2.2]oct-1-yl, Bicyclo[2.2.2]oct-2-yl, Bicyclo[3.3.0]octyl, Bicyclo[4.4.0]decyl und dergleichen.

Der Ausdruck "Aryl" umfasst im Rahmen der vorliegenden Erfindung ein- oder mehrkernige aromatische Kohlenwasserstoffreste, die unsubstituiert oder substituiert sein können. Aryl steht vorzugsweise für unsubstituiertes oder substituiertes Phenyl, Naphthyl, Indenyl, Fluorenyl, Anthracenyl, Phenanthrenyl, Naphthacenyl, Chrysenyl, Pyrenyl, etc., und besonders bevorzugt für Phenyl oder Naphthyl. Substituierte Aryle können in Abhängigkeit von der Anzahl und Größe ihrer Ringsysteme einen oder mehrere (z. B. 1, 2, 3, 4, 5 oder mehr als 5) Substituenten aufweisen. Diese sind vorzugsweise unabhängig voneinander ausgewählt unter Alkyl, Alkoxy, Cycloalkyl, Heterocycloalkyl, Aryl, Hetaryl, Halogen, Hydroxy, Mercapto, COOH, Carboxylat, SO₃H, Sulfonat, NE⁵E⁶, Nitro und Cyano, wobei E⁵ und E⁶ unabhängig voneinander für Wasserstoff, Alkyl, Cycloalkyl, Heterocycloalkyl, Aryl oder Hetaryl stehen. Halogensubstituenten sind vorzugsweise Fluor, Chlor oder Brom. Besonders bevorzugt steht Aryl für Phenyl, das im Falle einer Substitution im Allgemeinen 1, 2, 3, 4 oder 5, vorzugsweise 1, 2 oder 3 Substituenten tragen kann.

Aryl, das einen oder mehrere Reste trägt, steht beispielsweise für 2-, 3- und 4-Methylphenyl, 2,4-, 2,5-, 3,5- und 2,6-Dimethylphenyl, 2,4,6-Trimethylphenyl, 2-, 3-und 4-Ethylphenyl, 2,4-, 2,5-, 3,5- und 2,6-Diethylphenyl, 2,4,6-Triethylphenyl, 2-, 3-und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl; 2-, 3-und 4-Methoxyphenyl, 2,4-, 2,5-, 3,5- und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl und 2-, 3- und 4-Butoxyphenyl; 2-, 3- und 4-Cyanophenyl.

Der Ausdruck "Heterocycloalkyl" umfasst im Rahmen der vorliegenden Erfindung nichtaromatische, ungesättigte oder vollständig gesättigte, cycloaliphatische Gruppen mit im Allgemeinen 5 bis 8 Ringatomen, vorzugsweise 5- oder 6 Ringatomen, in denen 1, 2 oder 3 der Ringkohlenstoffatome durch Heteroatome, ausgewählt unter Sauerstoff, Stickstoff, Schwefel und einer Gruppe -NRe- ersetzt sind und das unsubstituiert ist oder mit einer oder mehreren, beispielsweise 1, 2, 3, 4, 5 oder 6 C₁-C₆-Alkylgruppen substituiert ist. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Dihydrothien-2-yl, Tetrahydrofuranyl, Dihydrofuran-2-yl, Tetrahydropyranyl, 1,2-Oxazolin-5-yl, 1,3-Oxazolin-2-yl und Dioxanyl genannt.

Der Ausdruck "Heteroaryl" umfasst im Rahmen der vorliegenden Erfindung unsubstituierte oder substituierte, heteroaromatische, ein- oder mehrkemige Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, wobei diese heterocycloaromatischen Gruppen im Falle einer Substitution im Allgemeinen 1, 2 oder 3 Substituenten, tragen können. Die Substituenten sind vorzugsweise ausgewählt unter C₁-C₆-Alkyl, C₁-C₆-Alkoxy, Hydroxy, Carboxy, Halogen und Cyano.

Stickstoffhaltige 5- bis 7-gliedrige Heterocycloalkyl- oder Heteroarylreste, die gegebenenfalls weitere unter Sauerstoff und Schwefel ausgewählte Heteroatome enthalten, umfassen beispielsweise Pyrrolyl, Pyrazolyl, Imidazolyl, Triazolyl, Pyrrolidinyl, Pyrazolinyl, Pyrazolidinyl, Imidazolinyl, Imidazolidinyl, Pyridinyl, Pyridazinyl, Pyrimidinyl, Pyrazinyl, Triazinyl, Piperidinyl, Piperazinyl, Oxazolyl, Isooxazolyl, Thiazolyl, Isothiazolyl, Indolyl, Chinolinyl, Isochinolinyl oder Chinaldinyl.

Die obigen Erläuterungen zu den Ausdrücken Cycloalkyl, Aryl, Heterocycloalkyl und Hetaryl gelten entsprechend für die Ausdrücke Cycloalkoxy, Aryloxy, Heterocycloalkoxy und Hetaryloxy.

Der Ausdruck Acyl steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen NE¹E², NE³E⁴, NE⁵E⁶, NE⁷E⁸ stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Carbamoyl steht für Gruppen der Formel -CO-NE⁷E⁸. Eine spezielle Ausführung ist unsubstituiertes Carbamoyl -CO-NH₂. Bevorzugt sind weiter N-(C₁-C₆)-monoalkylierte und N,N-(C₁-C₆)-dialkylierte Carbamoylgruppen.

Sulfamino steht für Gruppen der Formel -NE⁹-SO₃R^{a}, worin E⁸ und Ra unabhängig voneinander für Wasserstoff, Alkyl, Cycloalkyl, Aryl oder Hetaryl stehen. Eine spezielle Ausführung ist unsubstituiertes Sulfamino -NH-SO₃H.

Sulfamid steht für Gruppen der Formel -NE¹⁰-SO₂-NE¹¹E¹², worin E¹⁰, E¹¹ und E¹² unabhängig voneinander für Wasserstoff, Alkyl, Cycloalkyl, Aryl oder Hetaryl stehen. Eine spezielle Ausführung ist unsubstituiertes Sulfamid -NH-SO₂-NH₂.

Amidino steht für Gruppen der Formel -C(=NE¹³)-NE¹⁴E¹⁵, worin E¹³, E¹⁴ und E¹⁵ unabhängig voneinander für Wasserstoff, Alkyl, Cycloalkyl, Aryl oder Hetaryl stehen. Eine spezielle Ausführung ist unsubstituiertes Amidino -C(=NH)-NH₂.

Halogen steht für Fluor, Chlor, Brom oder Iod.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört.

Ankondensierte (anellierte) Ringe können unsubstituiert oder substituiert sein. Substituierte ankondensierte Ringe weisen vorzugsweise 1, 2 oder 3, insbesondere 1 oder 2 Substituenten auf. Die Substituenten sind vorzugsweise ausgewählt sind unter Alkyl, Alkoxy, Fluor, Chlor, COOH, Carboxylat, SO₃H, Sulfonat, NE⁵E⁶, Nitro, Alkylcarbonyloxy, Formyl, Acyl und Cyano.

M⁺ steht für ein Kationäquivalent, d. h. für ein einwertiges Kation oder den einer positiven Einfachladung entsprechenden Anteil eines mehrwertigen Kations. Bevorzugt sind Alkalimetallkationen, insbesondere Na⁺-, K⁺- oder Li⁺-Ionen oder Ammoniumionen.

Konkrete Beispiele für Reste R¹, R², R³ und R⁴, die nicht für Wasserstoff, CN oder für einen Phthalimidrest der allgemeinen Formel (II) stehen, sowie für Reste R⁵, R⁶, R⁷ und R⁸, die nicht für Wasserstoff oder CN stehen, sind im Einzelnen:
Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Hexadecyl, n-Octadecyl und n-Eicosyl, 2-Methoxyethyl, 2-Ethoxyethyl, 2-Propoxyethyl, 2-Butoxyethyl, 3-Methoxypropyl, 3-Ethoxypropyl, 3-Propoxypropyl, 3-Butoxypropyl, 4-Methoxybutyl, 4-Ethoxybutyl, 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Butylthio-ethyl, 3-Methylthiopropyl, 3-Ethylthiopropyl, 3-Propylthiopropyl, 3-Butylthiopropyl, 4-Methylthiobutyl, 4-Ethylthiobutyl, 4-Propylthiobutyl, 3,6-Dithiaheptyl, 3,6-Dithiaoctyl, 4,8-Dithianonyl, 3,7-Dithiaoctyl, 3,7-Di-thianonyl, 2- und 4-Butylthiobutyl, 4,8-Dithiadecyl, 3,6,9-Trithiadecyl, 3,6,9-Trithia-undecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3-Dimethyl-aminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Di-azaoctyl, 3,6-Dimethyl-3,6-diazaoctyl, 9-Methyl-3,6,9-triaza-decyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfinylethyl, 2-Ethylsulfinylethyl, 2-Propylsulfinylethyl, 2-Isopropylsulfinylethyl, 2-Butylsulfinylethyl, 2- und 3-Methylsulfinylpropyl, 2- und 3-Ethylsulfinylpropyl, 2- und 3-Propylsulfinylpropyl, 2- und 3-Butylsulfinylpropyl, 2- und 4-Methylsulfinylbutyl, 2- und 4-Ethylsulfinylbutyl, 2- und 4-Propylsulfinylbutyl und 4-Butylsulfinylbutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxy-tetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 3- und 4-Hydroxybutyl und 8-Hydroxy-4-oxaoctyl;
2-Cyanoethyl, 3-Cyanopropyl, 3- und 4-Cyanobutyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Butoxy, Pentoxy und Hexoxy;
Methylthio, Ethylthio, Propylthio, Butylthio, Pentylthio und Hexylthio;
Ethinyl, 1- und 2-Propinyl, 1-, 2- und 3-Butinyl, 1-, 2-, 3- und 4-Pentinyl, 1-, 2-, 3-, 4-und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16-und 17-Octadecinyl;
Ethenyl, 1- und 2-Propenyl, 1-, 2- und 3-Butenyl, 1-, 2-, 3- und 4-Pentenyl, 1-, 2-, 3-, 4-und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16-und 17-Octadecenyl;
Methylamino, Ethylamino, Propylamino, Butylamino, Pentylamino, Hexylamino, Dicyc-Iopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N-Dodecylaminosulfonyl, N,N-Diphenylaminosulfonyl, und N,N-Bis(4-Chlorphenyl)aminosulfonyl;
Methoxycarbonyl, Ethoxycarbonyl, Propoxycarbonyl Hexoxycarbonyl, Dodecyloxycarbonyl, Octadecyloxycarbonyl, Phenoxycarbonyl, (4-tert-Butylphenoxy)carbonyl und (4-Chlorphenoxy)carbonyl;
Methoxysulfonyl, Ethoxysulfonyl, Propoxysulfonyl, Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Fluor, Chlor und Brom;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methyl-cycloheptyl, 2-, 3- und 4-Ethylcycloheptyl, 3- und 4-Propylcycloheptyl, 3- und 4-lso-propylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4-und 5-Ethylcyclooctyl und 3-, 4- und 5-Propylcyclooctyl; 3- und 4-Hydroxycyclohexyl, 3- und 4-Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methyliso-indolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroiso-chinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethyl-chinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3-und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxy-phenyl; 2-, 3- und 4-Chlorphenyl und 2,4-, 3,5- und 2,6-Dichlorphenyl; 2-, 3-und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylamino-phenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)amino-phenyl, 3- und 4-(4-Pyridyl)aminophenyl, 3- und 4-(2-Pyrimidyl)-aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthyl-azo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Bevorzugte fluorhaltige Reste R¹ bis R⁸ sind die Folgenden:
2,2,2-Trifluorethyl, 2,2,3,3,3-Pentafluorpropyl, 2,2-Difluorethyl, 2,2,3,3,4,4,4-Heptafluorbutyl, 2,2,3,3,3-Pentafluorpropyl, 1H,1H-Pentadecafluoroctyl, 3-Brom-3,3-difluorpropyl, 3,3,3-Trifluorpropyl, 3,3,3-Trifluorpropyl, 1H, 1H,2H,2H-Pertluorodecyl, 3-(Perfluoroctyl)propyl, 4,4-Difluorbutyl-, 4,4,4-Trifluorbutyl, 5,5,6,6,6-Pentafluorhexyl, 2,2-Difluorpropyl, 2,2,2-Trifluor-1-phenylethylamino, 1-Benzyl-2,2,2-trifluorethyl, 2-Brom-2,2-difluorethyl, 2,2,2-Trifluor-1-pyridin-2-ylethyl, 2,2-Difluorpropyl, 2,2,2-Trifluor-1-(4-methoxyphenyl)ethylamino, 2,2,2-Trifluor-1-phenylethyl, 2,2-Difluor-1-phenylethyl, 1-(4-Brom-phenyl)-2,2,2-trifluorethyl, 3-Brom-3,3-difluorpropyl, 3,3,3-Trifluorpropylamin, 3,3,3-Trifluor-n-propyl, 1H,1H,2H,2H-Perfluordecyl, 3-(Perfluorctyl)propyl, Pentafluorphenyl, 2,3,5,6-Tetrafluorphenyl, 4-Cyano-(2,3,5,6)-Tetrafluorphenyl, 4-Carboxy-2,3,5,6-Tetrafluorphenyl, 2,4-Difluorphenyl, 2,4,5-Trifluorphenyl, 2,4,6-Trifluorphenyl, 2,5-Difluorphenyl, 2-Fluor-5-Nitrophenyl, 2-Fluor-5-trifluormethylphenyl, 2-Fluor-5-methylphenyl, 2,6-Difluorphenyl, 4-Carboxamido-2,3,5,6-tetrafluorphenyl, 2-Brom-4,6-difluorphenyl, 4-Brom-2-fluorphenyl, 2,3-Difluorphenyl, 4-Chlor-2-fluorphenyl, 2,3,4-Trifluorphenyl, 2-Fluor-4-iodphenyl, 4-Brom-2,3,5,6-tetrafluorphenyl; 2,3,6-Trifluorphenyl, 2-Brom-3,4,6-trifluorphenyl, 2-Brom-4,5,6-trifluorphenyl, 4-Brom-2,6-difluorphenyl, 2,3,4,5-Tetrafluorphenyl, 2,4-Difluor-6-nitrophenyl, 2-Fluor-4-nitrophenyl, 2-Chlor-6-fluorphenyl, 2-Fluor-4-methylphenyl, 3-Chlor-2,4-difluorphenyl, 2,4-Dibrom-6-fluorphenyl, 3,5-Dichlor-2,4-difluorphenyl, 4-Cyano-2-fluorphenyl, 2-Chlor-4-fluorphenyl, 2-Fluor-3-trifluormethylphenyl, 2-Trifluormethyl-6-fluorphenyl, 2,3,4,6-Tetrafluorphenyl, 3-Chlor-2-fluorphenyl, 5-Chlor-2-fluorphenyl, 2-Brom-4-chlor-6-fluorphenyl, 2,3-Dicyano-4,5,6-trifluorphenyl, 2,4,5-Trifluor-3-carboxyphenyl, 2,3,4-Trifluor-6-carboxyphenyl, 2,3,5-Trifluorphenyl, 4-Trifluormethyl2,3,5,6-tetrafluorphenyl, 2-Fluor-5-carboxyphenyl, 2-Chlor-4,6-Difluorphenyl, 6-Brom-3-chlor-2,4-difluorphenyl, 2,3,4-Trifluor-6-nitrophenyl, 2,5-Difluor-4-cyanophenyl, 2,5-Difluor-4-trifluormethylphenyl, 2,3-Difluor-6-nitrophenyl, 4-Trifluormethyl-2,3-difluorphenyl, 2-Brom-4,6-difluorphenyl, 4-Brom-2-fluorphenyl, 2-Nitrotetrafluorphenyl, 2,2',3,3',4',5,5',6,6'-Nonafluorbiphenyl, 2-Nitro-3,5,6-trifluorphenyl, 2-Brom-6-fluorphenyl, 4-Chlor-2-fluor-6-iodphenyl, 2-Fluor-6-carboxyphenyl, 2,4-Difluor-3-trifluorphenyl, 2-Fluor-4-trifluorphenyl, 2-Fluor-4-carboxyphenyl, 4-Brom-2,5-difluorphenyl, 2,5-Dibrom-3,4,6-trifluorphenyl, 2-Fluor-5-methylsulphonylpenyl, 5-Brom-2-fluorphenyl, 2-Fluor-4-hydroxymethylphenyl, 3-Fluor-4-brommethylphenyl, 2-Nitro-4-trifluormethylphenyl, 4-Trifluormethylphenyl, 2-Brom-4-trifluormethylphenyl, 2-Brom-6-Chlor-4-(trifluormethyl)phenyl, 2-Chlor-4-trifluormethylphenyl, 3-Nitro-4-(trifluormethyl)phenyl, 2,6-Dichlor-4-(trifluormethyl)phenyl, 4-Trifluorphenyl, 2,6-Dibrom-4-(trifluormethyl)phenyl, 4-Trifluormethyl-2,3,5,6-tetrafluorphenyl, 3-Fluor-4-trifluormethylphenyl, 2,5-Difluor-4-trifluormethylphenyl, 3,5-Difluor-4-trifluormethylphenyl, 2,3-Difluor-4-trifluormethylphenyl, 2,4-Bis(trifluormethyl)phenyl, 3-Chlor-4-trifluormethylphenyl, 2-Brom-4,5-di(trifluormethyl)phenyl, 5-Chlor-2-nitro-4-(trifluormethyl)phenyl, 2,4,6-Tris(trifluormethyl)phenyl, 3,4-Bis(trifluormethyl)phenyl, 2-Fluor-3-trifluormethylphenyl, 2-Iod-4-trifluormethylphenyl, 2-Nitro-4,5-bis(trifluormethyl)phenyl, 2-Methyl-4-(trifluormethyl)phenyl, 3,5-Dichlor-4-(trifluormethyl)phenyl, 2,3,6-Trichlor-4-(trifluormethyl)phenyl, 4-(Trifluormethyl)benzyl, 2-Fluor-4-(trifluormethyl)benzyl, 3-Fluor-4-(trifluormethyl)benzyl, 3-Chlor-4-(trifluormethyl)benzyl, 4-Fluorphenethyl, 3-(Trifluormethyl)phenethyl, 2-Chlor-6-fluorphenethyl, 2,6-Dichlorphenethyl, 3-Fluorphenethyl, 2-Fluorphenethyl, (2-Trifluormethyl)phenethyl, 4-Fluorphenethyl, 3-Fluorphenethyl, 4-Trifluormethylphenethyl, 2,3-Difluorphenethyl, 3,4-Difluorphenethyl, 2,4-Difluorphenethyl, 2,5-Difluorphenethyl, 3,5-Difluorphenethyl, 2,6-Difluorphenethyl,4-(4-Fluorphenyl)phenethyl, 3,5-Di(trifluormethyl)phenethyl, Pentafluorphenethyl, 2,4-Di(trifluormethyl)phenethyl, 2-Nitro-4-(trifluormethyl)phenethyl, (2-Fluor-3-trifluormethyl)phenethyl, (2-Fluor-5-trifluormethyl)phenethyl, (3-Fluor-5-trifluormethyl)phenethyl, (4-Fluor-2-trifluormethyl)phenethyl, (4-Fluor-3-trifluormethyl)phenethyl, (2-Fluor-6-trifluormethyl)phenethyl, (2,3,6-Trifluor)phenethyl, (2,4,5-Trifluor)phenethyl, (2,4,6-Trifluor)phenethyl, (2,3,4-Trifluor)phenethyl, (3,4,5-Trifluor)phenethyl, (2,3,5-Trifluor)phenethyl, (2-Chlor-5-fluor)phenethyl, (3-Fluor-4-trifluormethyl)phenethyl, (2-Chlor-5-trifluormethyl)phenethyl, (2-Fluor-3-chlor-5-trifluormethyl)phenethyl, (2-Fluor-3-chlor)phenethyl, (4-Fluor-3-chlor)phenethyl, (2-Fluor-4-chlor)phenethyl, (2,3-Difluor-4-methyl)phenethyl-, 2,6-Difluor-3-chlorphenethyl, (2,6-Difluor-3-methyl)phenethyl, (2-Trifluormethyl-5-chlor)phenethyl, (6-Chlor-2-fluor-5-methyl)phenethyl, (2,4-Dichlor-5-fluor)phenethyl, 5-Chlor-2-fluorphenethyl, (2,5-Difluor-6-chlor)phenethyl, (2,3,4,5-Tetrafluor)phenethyl, (2-Fluor-4-trifluormethyl)phenethyl, 2,3-(Difluor-4-trifluormethyl)phenethyl, (2,5-Di(trifluormethyl))phenethyl, 2-Fluor-3,5-dibromphenethyl, (3-Fluor-4-nitro)phenethyl, (2-Brom-4-trifluormethyl)phenethyl, 2-(Brom-5-fluor)phenethyl, (2,6-Difluor-4-brom)phenethyl, (2,6-Difluor-4-chlor)phenethyl, (3-Chlor-5-fluor)phenethyl, (2-Brom-5-trifluormethyl)phenethyl und dergleichen.

In einer speziellen Ausführung steht in den Chinonderivaten der Formel (I) keiner der Reste R¹ bis R⁴ für Halogen.

In einer weiteren speziellen Ausführung sind in den Chinonderivaten der Formel (I) die Reste R¹ bis R⁴ nur ausgewählt unter CN und Phthalimidresten der allgemeinen Formel (II).

In einer weiteren speziellen Ausführung stehen in den Chinonderivaten der Formel (I) zwei der Reste R¹ bis R⁴ für Cyano. Speziell stehen dann R¹ und R² bzw. R³ und R⁴ für Cyano.

In einer weiteren speziellen Ausführung stehen in den Chinonderivaten der Formel (I) zwei der Reste R¹ bis R⁴ für einen Phthalimidrest der allgemeinen Formel (II). Speziell stehen dann R¹ und R² bzw. R³ und R⁴ für einen Phthalimidrest der allgemeinen Formel (II).

Vorzugsweise weisen die Chinonderivate der allgemeinen Formel (I) wenigstens einen Phthalimidrest der allgemeinen Formel (II) auf, worin keiner der Reste R⁵ bis R⁸ für Halogen steht. In einer speziellen Ausführung weisen die Chinonderivate der allgemeinen Formel (I) ausschließlich Phthalimidreste der allgemeinen Formel (II) auf, worin keiner der Reste R⁵ bis R⁸ für Halogen steht.

Vorzugsweise weisen die Chinonderivate der allgemeinen Formel (I) wenigstens einen Phthalimidrest der allgemeinen Formel (II) auf, worin die Reste R⁵ bis R⁸ für Wasserstoff stehen. In einer speziellen Ausführung weisen die Chinonderivate der allgemeinen Formel (I) ausschließlich Phthalimidreste der allgemeinen Formel (II) auf, worin die Reste R⁵ bis R⁸ für Wasserstoff stehen.

Ein bevorzugtes Chinonderivat (I) ist die Verbindung der allgemeinen Formel (I.A)

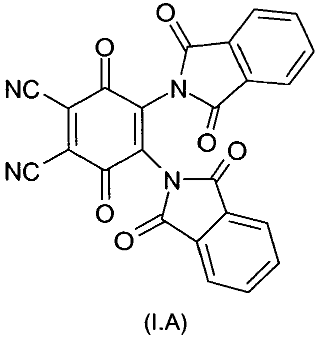

Geeignete Chinonverbindungen, die sich als Edukte zur Herstellung der erfindungsgemäßen und erfindungsgemäß verwendeten Chinonderivat (I) eignen, sind kommerziell erhältlich oder sind durch dem Fachmann bekannte Verfahren erhältlich. Geeignet als Edukte sind beispielsweise die entsprechenden halogenierten, speziell chlorierten, Chinone, wie das 2,3-Dichlor-5,6-dicyan-p-benzochinon (DDQ). So gelingt beispielsweise die Synthese der Verbindung (I.A) durch Umsetzung von DDQ mit Kaliumphthalimid nach folgendem Schema 1:

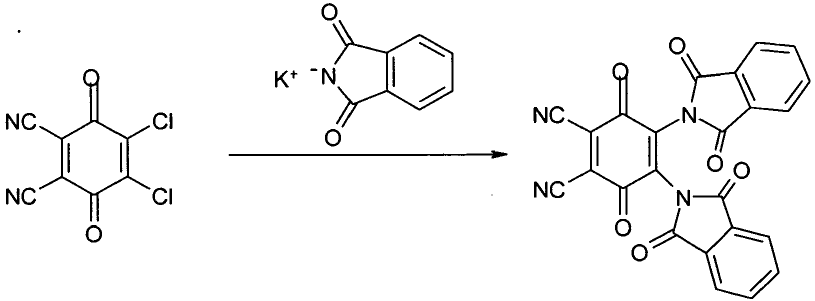

Ein weiterer Gegenstand der Erfindung ist daher ein Verfahren zur Herstellung von Chinonderivaten der allgemeinen Formel (I) 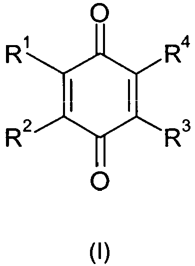 wobei R¹ bis R⁴ wie zuvor definiert sind,
bei dem man eine Verbindung der Formel (I), bei der wenigstens einer der Reste R¹ bis R⁴ für Cl oder Br steht, einer Umsetzung mit einer Phthalimidverbindung der allgemeinen Formel (II.a) 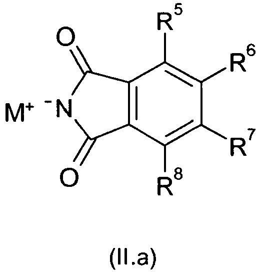 unterzieht, worin
M⁺ für ein Kationäquivalent steht,
R⁵, R⁶, R⁷ und R⁸ wie zuvor definiert sind.

Vorzugsweise wird zur Umsetzung mit der Phthalimidverbindung der allgemeinen Formel (II.a) eine Verbindung der Formel (I) eingesetzt, bei der die Halogenreste R¹ bis R⁴ für Cl stehen.

Die Herstellung der Phthalimid-Salze (II.a) erfolgt ausgehend von dem entsprechenden Phthalimid durch Umsetzung mit einer Base, vorzugsweise einem Alkalimetall- oder Erdalkalimetallhydroxid. Geeignete Alkalimetall- oder Erdalkalimetallhydroxide sind z. B. NaOH, KOH, Ca(OH)₂, etc. In einer geeigneten Ausführung wird eine alkoholische Alkalimetallhydroxidlösung eingesetzt.

Die Herstellung der Chinonderivate der allgemeinen Formel (I) erfolgt im Allgemeinen in Gegenwart eines unter den Reaktionsbedingungen inerten Lösungsmittels. Dazu zählen aromatische Lösungsmittel, wie Toluol oder Xylol, oder polare aprotische Lösungsmittel. Geeignete polare aprotische Lösungsmittel sind Acetonitril, Dimethylformamid, Dimethylacetamid, Dimethylsulfoxid und Stickstoffheterocyclen, wie Pyridin, Pyrimidin, Chinolin, Isochinolin, Chinaldin, N-Methylpiperidin, N-Methylpiperidon und N-Methylpyrrolidon. Geeignet sind auch Mischungen der zuvor genannten Lösungsmittel.

Die Reaktionstemperatur beträgt im Allgemeinen bei Umgebungstemperatur bis 200 °C, vorzugsweise 40 °C bis 160 °C, besonders bevorzugt 50 °C bis 150 °C.

Vorzugsweise liegt das Molmengenverhältnis von Phthalimid-Anion zur Anzahl der zu substituierenden Halogenatome bei etwa 1 : 1 (d. h. z. B. etwa 0,9 : 1 bis 1,1 : 1). Somit gelingt es in der Regel auch, gewünschtenfalls nur einen Teil der Halogenatome gegen Phthalimidgruppen auszutauschen.

Gewünschtenfalls können die Chinonderivate (I) wenigstens einem Aufarbeitungsschritt unterzogen werden. Dazu zählt die Reinigung nach üblichen, dem Fachmann bekannten Verfahren, z. B. durch Kristallisation oder Sublimation. Für einen Einsatz der Produkte als Dotierstoff, z. B. für Halbleitermaterialien, kann es von Vorteil sein, die Produkte einer Aufreinigung durch Sublimation zu unterziehen.

Die erfindungsgemäßen Chinonderivate der allgemeinen Formel (I) zeichnen sich durch wenigstens eine der folgenden vorteilhaften Eigenschaften aus:
- gute Zugängigkeit durch einfache Syntheseverfahren,
- hohe thermische Stabilität,
- gute Sublimierbarkeit,
- geringe Neigung zur Migration (Diffusion) des Dotierstoffs in benachbarte undotierte Schichten,
- homogene Verteilung des Dotierstoffs in dem damit dotierten Material,
- hohe Elektronenaffinität beim Einsatz als p-Dotierstoff,
- lange Lebensdauer der daraus hergestellten elektronischen Bauelemente, speziell OLEDs.

Die erfindungsgemäßen Chinonderivate der allgemeinen Formel (I) eignen sich hervorragend als Dotierstoff in der organischen Elektronik. Sie dienen speziell als Dotierstoff für organische halbleitende Materialien. Organische halbleitende Materialien, die wenigstens ein erfindungsgemäßes Chinonderivate der allgemeinen Formel (I) enthalten, eignen sich vorteilhaft zur Herstellung elektronischer Bauelemente. Dazu zählen vor allem organische Leuchtdioden (organische lichtemittierende Dioden, organic light emitting diods, OLED), organische Feldeffekttransistoren (Organic Field-Effect Transistors, OFET) sowie die organische Photovoltaik.

Die erfindungsgemäßen Chinonderivate der allgemeinen Formel (I) eignen sich speziell als p-Dotierstoff für ein Lochleitermaterial.

Die Wirkung der Dotierung auf ein Halbleitermaterial lässt sich z. B. mittels UPS (ultraviolet photoelectron spectroscopy) messen. Diese lässt z. B. Aussagen über den Einfluss der Dotierung auf die Energiedifferenz zwischen Fermi-Niveau und HOMO-Niveau zu.

Die erfindungsgemäßen Chinonderivate der allgemeinen Formel (I) eignen sich auch als Dotierstoff für Ladungstransportmaterialien.

Geeignete p-dotierbare Materialien, die mit wenigstens einem erfindungsgemäßen Chinonderivat der allgemeinen Formel (I) dotiert werden können, sind prinzipiell die dem Fachmann bekannten organischen Donor-Halbleitermaterialien. Dazu zählen:
Phthalocyanine, die nicht halogeniert oder halogeniert sind. Dazu zählen metallfreie oder zweiwertige Metalle oder metallatomhaltige Gruppen enthaltende Phthalocyanine, insbesondere die des Titanyloxy, Vanadyloxy, Eisens, Kupfers, Zinks, etc. Geeignete Phthalocyanine sind insbesondere Kupferphthalocyanin, Zinkphthalocyanin und metallfreies Phthalocyanin. Geeignete halogenierte Phthalocyanine sind z. B.
2,6,10,14-Tetrafluorphthalocyanine, z. B. 2,6,10,14-Tetrafluorkupferphthalocyanin und 2,6,10,14-Tetrafluorzinkphthalocyanin;
1,5,9,13-Tetrafluorphthalocyanine, z. B. 1,5,9,13-Tetrafluorkupferphthalocyanin und 1,5,9,13-Tetrafluorzinkphthalocyanin;
2,3,6,7,10,11,14,15-Octafluorophthalocyanin, z. B. 2,3,6,7,10,11,14,15-Octafluorokupferphtahlocyanin und 2,3,6,7,10,11,14,15-Octafluorozinkphthalocyanin.

Porphyrine, wie z. B. 5,10,15,20-Tetra(3-pyridyl)porphyrin (TpyP), oder aber Tetrabenzoporphyrine, wie beispielsweise metallfreies Tetrabenzoporphyrin, Kupfertetrabenzoporphyrin oder Zinktetrabenzoporphyrin.

Acene, wie Anthracen, Tetracen, Pentacen, die jeweils unsubstituiert oder substituiert sein können. Substituierte Acene umfassen vorzugsweise wenigstens einen Substituenten, der ausgewählt ist unter elektronenschiebenden Substituenten (z. B. Alkyl, Alkoxy, Ester, Carboxylat oder Thioalkoxy), elektronenziehenden Substituenten (z. B. Halogen, Nitro oder Cyano) und Kombinationen davon. Dazu zählen 2,9-Dialkylpentacene und 2,10-Dialkylpentacene, 2,10-Dialkoxypentacene, 1,4,8,11-Tetraalkoxypentacene und Rubren (5,6,11,12-Tetraphenylnaphthacen). Geeignete substituierte Pentacene sind in US 2003/0100779 und US 6,864,396 beschrieben, worauf hier Bezug genommen wird. Ein bevorzugtes Acen ist Rubren.

Coronene, wie Hexabenzocoronen (HBC-PhC₁₂), Coronendiimide, oder Triphenylene, wie 2,3,6,7,10,11-Hexahexylthiotriphenylen (HTT₆), 2,3,6,7,10,11-Hexakis-(4-n-nonyl-phenyl)-triphenylen (PTP₉) oder 2,3,6,7,10,11-Hexakis-(undecyloxy)-triphenylen (HAT₁₁).

Thiophene, Oligothiophene und substituierte Derivate davon. Geeignete Oligothiophene sind Quaterthiophene, Quinquethiophene, Sexithiophene, α,ω-Di(C₁-C₈)-alkyloligothiophenes, wie α,ω-Dihexylquaterthiophene, α,ω-Dihexylquinquethiophene und α,ω-Dihexylsexithiophene, Poly(alkylthiophene), wie Poly(3-hexylthiophen), Bis(dithienothiophene), Anthradithiophene und Dialkylanthradithiophene, wie Dihexylanthradithiophen, Phenylene-Thiophen-(P-T)-Oligomere und Derivate davon, speziell α,ωAlkyl-substituierte Phenylen-Thiophen-Oligomere.

Geeignet sind weiterhin Verbindungen des Typs α,α'-Bis(2,2 dicyanovinyl)quinque-thiophen (DCV5T), (3-(4-Octylphenyl)-2,2'-bithiophen) (PTOPT), Poly3-(4'-(1,4,7-trioxaoctyl)phenyl)thiophen (PEOPT), (Poly(3-(2'-methoxy-5'-octylphenyl)thiophen)) (POMeOPT), Poly(3-octylthiophen) (P₃OT), Poly(pyridopyrazin-vinylen)-Polythiophen-Blends, wie EHH-PpyPz, Copolymere PTPTB, BBL, F₈BT, PFMO, siehe Brabec C., Adv. Mater., 2996, 18, 2884, (PCPDTBT) Poly[2,6-(4,4-bis-(2-ethylhexyl)-4H-cyclopenta[2,1b;3,4 b']-dithiophen)-4,7-(2,1,3-benzothiadiazol).

Paraphenylenvinylen und Paraphenylenvinylen enthaltende Oligomere oder Polymere, wie z. B. Polyparaphenylenvinylen, MEH-PPV (Poly(2-methoxy-5-(2'-ethylhexyloxy)-1,4-phenylenevinylen, MDMO-PPV (Poly(2-methoxy-5-(3',7'-dimethyloctyloxy)-1,4-phenylen-vinylen)), PPV, CN-PPV (mit verschiedenen Alkoxy-Derivaten).

Phenylenethinylen/Phenylenvinylen-Hybridpolymere (PPE-PPV).

Polyfluorene und alternierende Polyfluoren-Copolymere, wie z. B. mit 4,7-Dithien-2'-yl-2,1,3-benzothiadiazol, geeignet sind weiterhin Poly(9,9'-dioctylfluoren-co-benzo-thiomdiazol) (F₈BT), Poly(9,9'-dioctylfluoren-co-bis-N,N'-(4-butyl-phenyl)-bis-N,N'-phenyl-1,4-phenylendiamin (PFB).

Polycarbazole, d. h. Carbazol enthaltende Oligomere und Polymere.

Polyaniline, d. h. Anilin enthaltende Oligomere und Polymere.

Triarylamine, Polytriarylamine, Polycyclopentadiene, Polypyrrole, Polyfurane, Polysilole, Polyphosphole, TPD, CBP, Spiro-MeOTAD. Bevorzugt sind Triphenylaminderivate, speziell N,N'-Bis-1-(naphthyl)-N,N'-diphenyl-1,1'-biphenyl-4,4'-diamin (NPD) und 4,4'-Bis(carbazol-9-yl)biphenyl (CBP).

Weitere zur Dotierung geeignete Materialien, speziell organische Donor-Halbleitermaterialien, werden auch im Folgenden bei den einzelnen elektronischen Bauteilen genannt, worauf hier Bezug genommen wird.

Vorzugsweise beträgt das Gewichtsmengenverhältnis von Dotierstoff zu dotierendem Material gleich oder weniger als 1 : 1, besonders bevorzugt gleich oder weniger als 0,5 : 1, insbesondere gleich oder weniger als 0,1 : 1. Das Gewichtsmengenverhältnis von Dotierstoff zu dotierendem Material liegt speziell in einem Bereich von 0,000001 : 1 bis 2 : 1, spezieller in einem Bereich von 0,00001 : 1 bis 1 : 1, noch spezieller in einem Bereich von 0,0001 : 1 bis 0,1 : 1.

Die Dotierung des zu dotierenden Materials mit den erfindungsgemäß zu verwendenden Chinonverbindungen (I) kann durch eines oder eine Kombination der folgenden Verfahren erfolgen:
a) Mischverdampfung im Vakuum mit einer Quelle für das zu dotierende Material und einer für den Dotierstoff;
b) Sequentielles Deponieren des zu dotierenden Materials und des Dotierstoffs auf einem Substrat mit anschließender Eindiffusion des Dotierstoffs, insbesondere durch thermische Behandlung;
c) Dotierung einer Schicht des zu dotierenden Materials durch eine Lösung von Dotierstoff mit anschließendem Verdampfen des Lösungsmittels, insbesondere durch thermische Behandlung;
d) Oberflächendotierung einer zu dotierenden Materialschicht durch eine oberflächlich aufgebrachte Schicht von Dotierstoff;
e) Herstellung einer Lösung von zu dotierendem Material und Dotierstoff und anschließende Herstellung einer Schicht aus dieser Lösung mittels konventioneller Methoden wie beispielsweise Verdampfen des Lösungsmittels oder spin coating.

Auf diese Weise können p-dotierte organische Halbleiter hergestellt werden, die vielfältig einsetzbar sind.

Ein weiterer Gegenstand der Erfindung ist eine organische Leuchtdiode (OLED), enthaltend wenigstens ein Chinonderivat der allgemeinen Formel (I), wie zuvor definiert. Die organische Leuchtdiode enthält das Chinonderivat (I) vorzugsweise in einer Leiterschicht, speziell in einer Lochleiterschicht.

Eine typische organische Leuchtdiode enthält eine Anode (An), eine Kathode (Ka) und eine zwischen der Anode (An) und der Kathode (Ka) angeordnete lichtemittierende Schicht (E). Eine typische organische Leuchtdiode enthält zusätzlich gegebenenfalls mindestens eine Blockschicht für Löcher und/oder Excitonen.

Die erfindungsgemäße organische Leuchtdiode (OLED) weist somit vorzugsweise den folgenden Aufbau auf:
eine Anode (An), eine Kathode (Ka) und eine zwischen der Anode (An) und der Kathode (Ka) angeordnete lichtemittierende Schicht (E) sowie gegebenenfalls mindestens eine Blockschicht für Löcher/Excitonen.

Die erfindungsgemäße OLED kann z. B. - in einer bevorzugten Ausführungsform - aus den folgenden Schichten aufgebaut sein:
1. Anode
2. Lochleiterschicht
3. lichtemittierende Schicht
4. Blockschicht für Löcher/Excitonen
5. Elektronenleiterschicht
6. Kathode

Es sind auch von dem vorstehend genannten Aufbau verschiedene Schichtenfolgen möglich, die dem Fachmann bekannt sind. Beispielsweise ist es möglich, dass die OLED nicht alle der genannten Schichten aufweist, z. B. ist eine OLED mit den Schichten (1) (Anode), (3) (lichtemittierende Schicht) und (6) (Kathode) ebenfalls geeignet, wobei die Funktionen der Schichten (2) (Lochleiterschicht) und (4) (Blockschicht für Löcher/Excitonen) und (5) (Elektronenleiterschicht) durch die angrenzenden Schichten übernommen werden. OLEDs, die die Schichten (1), (2), (3) und (6) bzw. die Schichten (1), (3), (4), (5) und (6) aufweisen, sind ebenfalls geeignet. Des Weiteren können die OLEDs zwischen der Anode (1) und der Lochleiterschicht (2) eine Blockschicht für Elektronen/Excitonen aufweisen.

Es ist des Weiteren möglich, dass mehrere der vorstehend genannten Funktionen (Elektronen-/Excitonenblocker, Loch-/Excitonenblocker, Loch-Injektion, Lochleitung, Elektroneninjektion, Elektronenleitung) in einer Schicht vereint sind und z. B. von einem einzigen in dieser Schicht vorliegenden Material übernommen werden. Beispielsweise kann ein in der Lochleiterschicht eingesetztes Material in einer Ausführungsform gleichzeitig Excitonen und/oder Elektronen blocken.

Des Weiteren können die einzelnen der vorstehend genannten Schichten der OLED wiederum aus zwei oder mehreren Schichten aufgebaut sein. Beispielsweise kann die Lochleiterschicht aus einer Schicht aufgebaut sein, in die aus der Elektrode Löcher injiziert werden, und einer Schicht, die die Löcher von der Loch-injizierenden Schicht weg in die lichtemittierende Schicht transportiert. Die Elektronenleitungsschicht kann ebenfalls aus mehreren Schichten bestehen, z. B. einer Schicht, worin Elektronen durch die Elektrode injiziert werden, und einer Schicht, die aus der Elektronen-Injektionsschicht Elektronen erhält und in die lichtemittierende Schicht transportiert.

In einer speziellen Ausführungsform enthält die erfindungsgemäße OLED eine Lochleiterschicht, die wenigstens ein erfindungsgemäßes Chinonderivat der Formel (I) als Dotierstoff enthält. Diese dotierte Lochleiterschicht kann als einzige Lochleiterschicht oder in Kombination mit wenigstens einer weiteren undotierten oder dotierten Lochleiterschicht eingesetzt werden.

Die genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die als Emittersubstanzen verwendeten Verbindungen angepasst ist.

Um besonders effiziente OLEDs zu erhalten, sollte z. B. das HOMO (höchstes besetztes Molekülorbital) der Lochleiterschicht mit der Arbeitsfunktion der Anode angeglichen sein, und das LUMO (niedrigstes unbesetztes Molekülorbital) der Elektronenleiterschicht sollte mit der Arbeitsfunktion der Kathode angeglichen sei, soweit die vorstehend genannten Schichten in den erfindungsgemäßen OLEDs vorliegen.

Die Anode (1) ist eine Elektrode, die positive Ladungsträger bereitstellt. Sie kann z. B. aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIIIa. Wenn die Anode lichtdurchlässig sein soll, werden im Allgemeinen gemischte Metalloxide der Gruppen IIb, IIIb und IVb des Periodensystems der Elemente (alte IUPAC-Version) eingesetzt, z. B. Indium-Zinn-Oxid (ITO). Es ist ebenfalls möglich, dass die Anode (1) ein organisches Material, z. B. Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können. Bevorzugt wird als Material für die Anode (1) ITO eingesetzt.

Geeignete Lochleitermaterialien für die Schicht (2) und gegebenenfalls weitere lochleitende Schichten der erfindungsgemäßen OLEDs sind neben den zuvor genannten z. B. in Kirk-Othmer Encyclopedia of Chemical Technologie, 4. Auflage, Vol. 18, Seiten 837 bis 860, 1996, offenbart. Sowohl Löcher-transportierende Moleküle als auch Polymere können als Lochtransportmaterial eingesetzt werden. Üblicherweise eingesetzte Löcher-transportierende Moleküle sind ausgewählt aus der Gruppe bestehend aus N,N'-Bis-1-(naphthyl)-N,N'-diphenyl-1,1'-biphenyl-4,4'-diamin (NPD) und 4,4'-Bis(carbazol-9-yl)biphenyl (CBP),Tris-[N-(1-naphthyl)-N-(phenylamino)]triphenylamin (1-NaphDATA), 4,4'-Bis[N-(1-naphthyl)-N-phenylamino]biphenyl (α-NPD), N,N'-Diphenyl-N,N'-bis(3-methylphenyl)-[1,1'-biphenyl]-4,4'-diamin (TPD), 1,1-Bis[(di-4-tolylamino)phenyl]cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenyl-hydrazon (DEH), Triphenylamin (TPA), Bis[4-(N,N-diethylamino)-2-methylphenyl)-(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB), 4,4',4"-tris(N,N-Diphenylamino)triphenylamin (TDTA), 4,4',4"-tri(N-carbazolyl)triphenylamine (TCTA), N,N'-Bis(naphthalen-2-yl)-N,N'-bis(phenyl)-benzidin (β-NPB), N,N'-Bis(3-methylphenyl)-N,N'-bis(phenyl)-9,9-spirobifluoren (Spiro-TPD), N,N'-Bis(naphthalen-1-yl)-N,N'-bis(phenyl)-9,9-spirobifluoren (Spiro-NPB), N,N'-Bis(3-methylphenyl)-N,N'-bis(phenyl)-9,9-dimethylfluoren (DMFL-TPD), N,N'-Bis(3-methylphenyl)-N,N'-bis(phenyl)-9,9-diphenylfluoren (DPFL-TPD), N,N'-Bis(naphthalen-1-yl)-N,N'-bis(phenyl)-9,9-diphenylfluoren (DPFL-NPB), 2,2',7,7'-Tetrakis(N,N-diephenylamino)-9,9'-spirobifluoren (Spiro-TAD), 9,9-Bis[4-N,N-bis-biphenyl-4-yl-amino)phenyl]-9H-fluoren (BPAPF), 9,9-Bis[4-(N,N-bis-naphthalen-2-yl-amino)phenyl]-9H-fluoren (NPAPF), 9,9-Bis[4-(N,N-bis-naphthalen-2-yl-N,N'-bis-phenyl-amino)phenyl]-9H-fluoren (NPBAPF), 2,2',7,7'-Tetrakis[N-naphthalenyl(phenyl)-amino]-9,9'-spirobifluoren (Spiro-2NPB), N,N'-bis(phenanthren-9-yl)-N,N'-bis(phenyl)-benzidin (PAPB), 2,7-Bis[N,N-bis(9,9-spiro-bifluoren-2-yl)-amino]-9,9-spirobifluoren (Spiro-5), 2,2'-Bis[N,N-bis(biphenyl-4-yl)amino]-9,9-spirobifluoren (2,2'-Spiro-DBP), 2,2'-Bis(N,N-di-phenyl-amino)-9,9-spirobifluoren (Spiro-BPA), 2,2',7,7'-tetra(N,N-di-Tolyl)amino-Spiro-bifluoren (Spiro-TTB), N,N,N',N'-tetra-naphthalen-2-yl-benzidin (TNB), Porphyrinverbindungen und Phthalocyaninen wie Kupferphthalocyanine. Üblicherweise eingesetzte Löcher-transportierende Polymere sind ausgewählt aus der Gruppe bestehend aus Polyvinylcarbazolen, (Phenylmethyl)polysilanen und Polyanilinen. Es ist ebenfalls möglich, Löcher-transportierende Polymere durch Dotieren Löcher-transportierender Moleküle in Polymere wie Polystyrol und Polycarbonat zu erhalten. Geeignete Löcher-transportierende Moleküle sind die bereits vorstehend genannten Moleküle.

Weiterhin können - in einer Ausführungsform - Carben-Komplexe als Lochleitermaterialien eingesetzt werden, wobei die Bandlücke des mindestens einen Lochleitermaterials im Allgemeinen größer ist als die Bandlücke des eingesetzten Emittermaterials. Dabei ist unter Bandlücke im Sinne der vorliegenden Anmeldung die Triplett-Energie zu verstehen. Geeignete Carben-Komplexe sind z. B. Carben-Komplexe, wie sie in WO 2005/019373 A2, WO 2006/056418 A2, WO 2005/113704, WO 2007/115970, WO 2007/115981 und WO 2008/000727 beschrieben sind. Ein Beispie! für einen geeigneten Carbenkomplex ist Ir(dpbic)₃ mit der Formel: 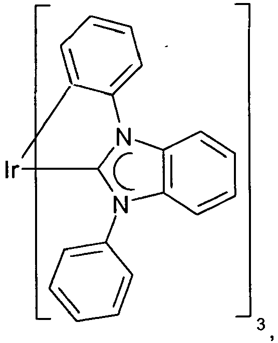 der z. B. in WO2005/019373 offenbart ist.

Die lichtemittierende Schicht (3) enthält mindestens ein Emittermaterial. Dabei kann es sich grundsätzlich um einen Fluoreszenz- oder Phosphoreszenzemitter handeln, wobei geeignete Emittermaterialien dem Fachmann bekannt sind. Bevorzugt handelt es sich bei dem mindestens einen Emittermaterial um einen Phosphoreszenzemitter. Die bevorzugt eingesetzten Phosphoreszenzemitter-Verbindungen basieren auf Metallkomplexen, wobei insbesondere die Komplexe der Metalle Ru, Rh, Ir, Pd und Pt, vor allem die Komplexe des Ir Bedeutung erlangt haben.

Geeignete Metallkomplexe zur Verwendung in den erfindungsgemäßen OLEDs sind z. B. in den Schriften WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981 und WO 2008/000727 beschrieben.

Weitere geeignete Metallkomplexe sind die kommerziell erhältlichen Metallkomplexe Tris(2-phenylpyridin)iridium(III), Iridium(III)tris(2-(4-tolyl)pyridinato-N,C^{2'}), Iridium(III)tris(1-phenylisochinolin), Iridium(III)bis(2-2'-benzo-thienyl)pyridinato-N,C^{3'})(acetylacetonat), Iridium(III)bis(2-(4,6-difluorophenyl)pyridinato-N,C²)picolinat, Iridium(III)bis(1-phenylisochinolin)(acetylacetaonat), Iridium(III)bis(di-benzo[f,h]chin-oxalin)(acetylacetaonat), Iridium(III)bis(2-methyldi-benzo[f,h]chinoxalin)(acetylacetonat) und Tris(3-methyl-1-phenyl-4-trimethyl-acetyl-5-pyrazolin)terbium(III).

Des Weiteren sind die folgenden kommerziell erhältlichen Materialien geeignet: Tris(dibenzoylacetonato)-mono(phenanthrolin)-europium(III), Tris(dibenzoylmethan)-mono(phenanthrolin)europium(III), Tris(dibenzoylmethan)-mono(5-aminophenan-throlin)europium(III), Tris(di-2-naphthoylmethan)-mono(phenanthrolin)-europium(III), Tris(4-bromobenzoylmethan)-mono(phenanthrolin)-europium(III), Tris(di-biphenyl-methan)-mono(phenanthrolin)-europium(III), Tris(dibenzoylmethan)-mono(4,7-diphenyl-phenanthrolin)-europium(III), Tris(dibenzoylmethan)-mono(4,7-di-methylphenanthrolin)-europium(III), Tris(dibenzoylmethan)-mono(4,7-dimethyl-phenanthrolin-disulfonsäure)-europium(III)-dinatriumsalz, Tris[di(4-(2-(2-ethoxy-ethoxy)ethoxy)benzoylmethan)]-mono(phenanthrolin)-europium(III) und Tris[di[4-(2-(2-ethoxyethoxy)ethoxy)benzoyl-methan)]mono-(5-aminophenanthrolin)-europium(III).

Geeignete Triplett-Emitter sind z. B. Carbenkomplexe. In einer Ausführungsform der vorliegenden Erfindung werden die Verbindungen der Formel (I) bzw. (Ib) in der lichtemittierenden Schicht als Matrixmaterial gemeinsam mit Carbenkomplexen als Triplett-Emitter eingesetzt. Geeignete Carbenkomplexe sind dem Fachmann bekannt und z. B. in WO 2005/019373 A2, WO 2006/056418 A2, WO 2005/113704, WO 2007/115970, WO 2007/115981 und WO 2008/000727 beschrieben.

Die lichtemittierende Schicht kann neben dem Emittermaterial weitere Komponenten enthalten. Beispielsweise kann in der lichtemittierenden Schicht ein fluoreszierender Farbstoff anwesend sein, um die Emissionsfarbe des Emittermaterials zu verändern. Des Weiteren kann - in einer bevorzugten Ausführungsform - ein Matrixmaterial eingesetzt werden. Dieses Matrixmaterial kann ein Polymer sein, z. B. Poly(N-vinylcarbazol) oder Polysilan. Das Matrixmaterial kann jedoch ebenfalls ein kleines Molekül sein, z. B. 4,4'-N,N'-Dicarbazolbiphenyl (CDP=CBP) oder tertiäre aromatische Amine wie z. B. TCTA.

Geeignete Metallkomplexe zur Verwendung als Matrixmaterialien und/oder Loch/Excitonen-Blockermaterialien in OLEDs sind z. B. auch Carben-Komplexe, wie sie in WO 2005/019373 A2, WO 2006/056418 A2, WO 2005/113704, WO 2007/115970, WO 2007/115981 und WO 2008/000727 beschrieben sind. Auf die Offenbarung der genannten WO-Anmeldungen wird hierbei explizit Bezug genommen.

Falls die OLED eine Blockschicht für Löcher aufweist, so weist diese üblicherweise in OLEDs eingesetzte Lochblockermaterialien auf, wie 2,6-Bis(N-carbazolyl)pyridine (mCPy), 2,9-Dimethyl-4,7-diphenyl-1,10-phenanthrolin (Bathocuproin, (BCP)), Bis-(2-methyl-8-chinolinato)-4-phenyl-phenylato)-aluminium(III) (BAIq), PhenothiazinS,S-dioxidderivate und 1,3,5-tris(N-Phenyl-2-benzylimidazol)-benzol) (TPBI), wobei TPBI auch als elektronenleitendes Material geeignet ist. In einer weiteren Ausführungsform können Verbindungen, die aromatische oder heteroaromatische über Carbonylgruppen enthaltende Gruppen verbundene Ringe enthalten, wie sie in WO2006/100298 offenbart sind, Disilylverbindungen, ausgewählt aus der Gruppe bestehend aus Disilylcarbazolen, Disilylbenzofuranen, Disilylbenzothiophenen, Disilylbenzophospholen, Disilylbenzothiophen-S-oxiden und Disilylbenzothiophen-S,S-dioxiden wie sie z. B. in den nicht vorveröffentlichten PCT-Anmeldungen mit den Aktenzeichen PCT/EP2008/058207 und PCT/EP2008/058106 genannt sind sowie Disilylverbindungen wie sie in W02008/034758 offenbart sind als Blockschicht für Löcher/- Excitonen (4) oder als Matrixmaterialien in der lichtemittierenden Schicht (3) eingesetzt werden.

In einer bevorzugten Ausführungsform betrifft die vorliegende Erfindung eine erfindungsgemäße OLED, umfassend die Schichten (1) Anode, (2) Lochleiterschicht, (3) lichtemittierende Schicht, (4) Blockschicht für Löcher/Excitonen, (5) Elektronenleiterschicht und (6) Kathode, sowie gegebenenfalls weitere Schichten, wobei die lochleitende Schicht mindestens eine Verbindung der Formel (I) enthält.

Geeignete Elektronenleitermaterialien für die Schicht (5) der erfindungsgemäßen OLEDs umfassen mit oxinoiden Verbindungen chelatisierte Metalle, wie 2,2',2"-(1,3,5-phenylen)tris-[1-phenyl-1H-benzimidazol] (TPBI), Tris(8-chinolinolato)aluminium (Alq₃), Verbindungen auf Phenanthrolinbasis wie 2,9-Dimethyl-4,7,-diphenyl-1,10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ), 8-Hydroxyquinolinolato-lithium (Liq), 4,7-Diphenyl-1,10-phenanthrolin (BPhen), Bis-(2-methyl-8-quinolinolat)-4-(phenylphenolato)aluminium (BAlq), 1,3-Bis(2-(2,2'-bipyridin-6-yl)-1,3,4-oxadiazo-5-yl]benzen (Bpy-OXD), 6,6'-Bis[5-(biphenyl-4-yl)-1,3,4-oxadiazo-2-yl]-2,2'-bipyridyl (BP-OXD-Bpy), 4-(Naphthalen-1-yl)-3,5-diphenyl-4H-1,2,4-triazol (NTAZ), 2,9-Bis(naphthalen-2-yl)-4,7-diphenyl-1,10-phenanthrolin (NBphen), 2,7-Bis[2-(2,2'-bipyridin-6-yl)-1,3,4-oxadiazo-5-yl]-9,9-dimethylfluoren (Bby-FOXD), 1,3-Bis[2-(4-tert-butylphenyl)-1,3,4-oxadiazo-5-yl]benzen (OXD-7), Tris(2,4,6-trimethyl-3-(pyridin-3-yl)phenyl)boran (3TPYMB), 1-Methyl-2-(4-(naphthalen-2-yl)phenyl)-1H-imidazo[4,5-f][1,10]phenanthrolin (2-NPIP), 2-Phenyl-9,10,-di(naphthalen-2-yl)-anthracen (PADN), 2-(Naphthalen-2-yl)-4,7-diphenyl-1,10,phenanthrolin (HNBphen). Dabei kann die Schicht (5) sowohl zur Erleichterung des Elektronentransports dienen als auch als Pufferschicht oder als Sperrschicht, um ein Quenchen des Excitons an den Grenzflächen der Schichten der OLED zu vermeiden. Vorzugsweise verbessert die Schicht (5) die Beweglichkeit der Elektronen und reduziert ein Quenchen des Excitons. In einer bevorzugten Ausführungsform wird TPB! als Elektronenleitermaterial eingesetzt.

Von den vorstehend als Lochleitermaterialien und Elektronenleitermaterialien genannten Materialien können einige mehrere Funktionen erfüllen. Zum Beispiel sind einige der Elektronen-leitenden Materialien gleichzeitig Löcher-blockende Materialien, wenn sie ein tief liegendes HOMO aufweisen. Diese können z. B. in der Blockschicht für Löcher/Excitonen (4) eingesetzt werden. Es ist jedoch ebenfalls möglich, dass die Funktion als Loch/Excitonenblocker von der Schicht (5) mit übernommen wird, so dass die Schicht (4) entfallen kann.

Auch die Elektronenleitermaterialien können dotiert sein. Diese Dotierung dient z. B. dazu, die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Die Elektronenleitermaterialien können z. B. mit Alkalimetallen dotiert werden, beispielsweise Alq₃ mit Lithium. Außerdem können Elektronenleiter mit Salzen wie Cs₂CO₃ dotiert werden. Die elektronische Dotierung ist dem Fachmann bekannt und z. B. in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo. Appl. Phys. Lett., Vol. 82, No. 25, 23 June 2003 und Pfeiffer et al., Organic Electronics 2003, 4, 89 - 103 offenbart.

Die Kathode (6) ist eine Elektrode, die zur Einführung von Elektronen oder negativen Ladungsträgern dient. Geeignete Materialien für die Kathode sind ausgewählt aus der Gruppe bestehend aus Alkalimetallen der Gruppe la, z. B. Li, Cs, Erdalkalimetallen der Gruppe IIa, z. B. Calcium, Barium oder Magnesium, Metallen der Gruppe IIb des Periodensystems der Elemente (alte IUPAC-Version), umfassend die Lanthaniden und Aktiniden, z. B. Samarium. Des Weiteren können auch Metalle wie Aluminium oder Indium, sowie Kombinationen aller genannten Metalle eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Die OLED gemäß der vorliegenden Erfindung kann zusätzlich weitere Schichten enthalten, die dem Fachmann bekannt sind. Beispielsweise kann zwischen der Schicht (2) und der lichtemittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der lichtemittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält die erfindungsgemäße OLED zusätzlich zu den Schichten (1) bis (6) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der lichtemittierenden Schicht (3);
- eine Elektronen-Injektionsschicht zwischen der Elektronen-transportierenden Schicht (5) und der Kathode (6).

Materialien für eine Lochinjektionsschicht können ausgewählt sein aus Kupferphthalocyanin, 4,4',4"-Tris(N-3-methylphenyl-N-phenyl-amino)triphenylamin (m-MTDATA), 4,4',4"-Tris(N-(2-naphthyl)-N-phenyl-amino)triphenylamin (2T-NATA), 4,4',4"-Tris(N-(1-naphthyl)-N-phenyl-amino)triphenylamin (1T-NATA), 4,4',4"-Tris(N,N-diphenylamino)triphenylamin (NATA), Titanoxidphtalocyanin, 2,3,5,6-Tetrafluoro-7,7,8,8-tetracyano-quinodimethan (F4-TCNQ), Pyrazino[2,3-f][1,10]phenanthrolin-2,3-dicarbonitril (PPDN), N,N,N',N'-Tetrakis(4-methoxyphenyl)benzidin (MeO-TPD), 2,7-Bis[N,N-bis(4-methoxy-phenyl)amino]-9,9-spirobifluoren (MeO-Spiro-TPD), 2,2'-Bis[N,N-bis(4-methoxy-phenyl)amino]-9,9-spirobifluoren (2,2'-MeO-Spiro-TPD), N,N'-di-phenyl-N,N'-di-[4-(N,N-di-tolyl-amino) phenyl]benzidin (NTNPB), N,N'-di-phenyl-N,N'-di-[4-(N,N-di-phenyl-amino) phenyl]benzidin (NPNPB), N,N'-di(naphthalen-2-yl)-N,N'-diephenylbenzen-1,4-diamin (β-NPP).

Als Material für die Elektroneninjektionsschicht kann beispielsweise LiF gewählt werden.

Dem Fachmann ist bekannt, wie er (z. B. auf Basis von elektrochemischen Untersuchungen) geeignete Materialien auswählen muss. Geeignete Materialien für die einzelnen Schichten sind dem Fachmann bekannt und z. B. in WO 00/70655 offenbart.

Des Weiteren ist es möglich, dass einige der in der erfindungsgemäßen OLED eingesetzten Schichten oberflächenbehandelt sind, um die Effizienz des Ladungsträgertransports zu erhöhen. Die Auswahl der Materialien für jede der genannten Schichten ist bevorzugt dadurch bestimmt, eine OLED mit einer hohen Effizienz und Lebensdauer zu erhalten.

Die Herstellung der erfindungsgemäßen OLED kann nach dem Fachmann bekannten Methoden erfolgen. Im Allgemeinen wird die erfindungsgemäße OLED durch aufeinanderfolgende Dampfabscheidung (Vapor Deposition) der einzelnen Schichten auf ein geeignetes Substrat hergestellt. Geeignete Substrate sind z. B. Glas, anorganische Halbleiter oder Polymerfilme. Zur Dampfabscheidung können übliche Techniken eingesetzt werden wie thermische Verdampfung, Chemical Vapor Deposition (CVD), Physical Vapor Deposition (PVD) und andere. In einem alternativen Verfahren können die organischen Schichten der OLED aus Lösungen oder Dispersionen in geeigneten Lösungsmitteln aufgetragen werden, wobei dem Fachmann bekannte Beschichtungstechniken angewendet werden.

Im Allgemeinen haben die verschiedenen Schichten folgende Dicken: Anode (1) 50 bis 500 nm, bevorzugt 100 bis 200 nm; Löcher-leitende Schicht (2) 5 bis 100 nm, bevorzugt 20 bis 80 nm, lichtemittierende Schicht (3) 1 bis 100 nm, bevorzugt 10 bis 80 nm, Blockschicht für Löcher/Excitonen (4) 2 bis 100 nm, bevorzugt 5 bis 50 nm, Elektronenleitende Schicht (5) 5 bis 100 nm, bevorzugt 20 bis 80 nm, Kathode (6) 20 bis 1000 nm, bevorzugt 30 bis 500 nm. Die relative Lage der Rekombinationszone von Löchern und Elektronen in den erfindungsgemäßen OLED in Bezug zur Kathode und somit das Emissionsspektrum der OLED können u. a. durch die relative Dicke jeder Schicht beeinflusst werden. Das bedeutet, die Dicke der Elektronentransportschicht sollte bevorzugt so gewählt werden, dass die Lage der Rekombinationszone auf die optische Resonatoreigenschaft der Diode und damit auf die Emissionswellenlänge des Emitters abgestimmt ist. Das Verhältnis der Schichtdicken der einzelnen Schichten in der OLED ist von den eingesetzten Materialien abhängig. Die Schichtdicken von gegebenenfalls eingesetzten zusätzlichen Schichten sind dem Fachmann bekannt.

Durch Einsatz der Chinonverbindungen der Formel (I) in einer Leiterschicht, speziell einer Lochleiterschicht, können OLEDs mit hoher Effizienz und Lebensdauer erhalten werden. Die Effizienz der OLEDs kann des Weiteren durch Optimierung der anderen Schichten der OLEDs verbessert werden. Beispielsweise können hoch effiziente Kathoden wie Ca oder Ba, gegebenenfalls in Kombination mit einer Zwischenschicht aus LiF, eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die OLEDs können weiterhin zumindest eine zweite lichtemittierende Schicht enthalten. Die Gesamtemission der OLEDs kann sich dabei aus der Emission der zumindest zwei lichtemittierenden Schichten zusammensetzen und kann auch weißes Licht umfassen.

Ein weiterer Gegenstand der vorliegenden Erfindung ist eine lochleitende Schicht, enthaltend mindestens ein Chinonderivat der Formel (I), wie zuvor definiert.

Die OLEDs können in allen Vorrichtungen eingesetzt werden, worin Elektrolumineszenz nützlich ist. Geeignete Vorrichtungen sind bevorzugt ausgewählt aus stationären und mobilen Bildschirmen und Beleuchtungseinheiten. Stationäre Bildschirme sind z. B. Bildschirme von Computern, Fernsehern, Bildschirme in Druckern, Küchengeräten sowie Reklametafeln, Beleuchtungen und Hinweistafeln. Mobile Bildschirme sind z. B. Bildschirme in Handys, Laptops, Digitalkameras, Fahrzeugen sowie Zielanzeigen an Bussen und Bahnen.

Weiterhin können die Chinonderivate der Formel (I) in OLEDs mit inverser Struktur eingesetzt werden. Der Aufbau von inversen OLEDs und die üblicherweise darin eingesetzten Materialien sind dem Fachmann bekannt.

Des Weiteren betrifft die vorliegende Erfindung eine Vorrichtung, ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen und Beleuchtungseinheiten, enthaltend mindestens eine erfindungsgemäße organische Leuchtdiode oder mindestens eine erfindungsgemäße lochleitende Schicht.

Die Chinonderivate der allgemeinen Formel (I) eignen sich auch besonders vorteilhaft als Dotierstoff für organische Halbleiter. Sie fungieren dabei in der Regel als Dotierstoff für p-Halbleiter.

Halbleitermaterialien, die mit wenigstens einem Chinonderivat der allgemeinen Formel (I) dotiert sind, verfügen über eine hohe Ladungstransportmobilität und/oder haben ein hohes on/off-Verhältnis. Sie eignen sich in besonders vorteilhafter Weise für organische Feldeffekttransistoren (OFETs).

Die erfindungsgemäßen Verbindungen und mit ihnen dotierte organische Halbleiter eignen sich vorteilhaft zur Herstellung von integrierten Schaltkreisen (ICs). Dazu zählen z. B. solche Schaltkreise, für die bislang übliche MOSFET (metal oxide semiconductor field-effect transistor (MOSFET)) zum Einsatz kommen. Dabei handelt es sich dann um CMOS-analoge Halbleiterbausteine, z. B. für Mikroprozessoren, Mikrocontroller, statische RAM, und andere digitale Logikbausteine.

Zur Herstellung von Halbleitermaterialien können die erfindungsgemäßen Verbindungen der Formel (I) bzw. die damit dotierten organischen Halbleiter nach einem der folgenden Verfahren weiterverarbeitet werden: Drucken (Offset, Flexo, Gravur, Screen, Inkjet, Elektrofotografie), Verdampfen, Lasertransfer, Fotolithografie, Dropcasting. Sie eignen sich insbesondere für einen Einsatz in Displays (speziell großflächigen und/oder flexiblen Displays) und RFID-Tags.

Gegenstand der Erfindung sind weiterhin organische Feldeffekttransistoren, umfassend ein Substrat mit wenigstens einer Gate-Struktur, einer Source-Elektrode und einer Drain-Elektrode und wenigstens einer Verbindung der Formel I, wie zuvor definiert, als Dotierstoff. Gegenstand der Erfindung sind weiterhin Substrate mit einer Vielzahl von organischen Feldeffekttransistoren, wobei zumindest ein Teil der Feldeffekttransistoren wenigstens eine Verbindung der Formel I, wie zuvor definiert, als Dotierstoff enthält. Gegenstand der Erfindung sind auch Halbleiterbausteine, die wenigstes ein solches Substrat umfassen.

Eine spezielle Ausführungsform ist ein Substrat mit einem Muster (Topographie) von organischen Feldeffekttransistoren, wobei jeder Transistor
- einen auf dem Substrat befindlichen organischen Halbleiter;
- eine Gate-Struktur zur Steuerung der Leitfähigkeit des leitenden Kanals; und
- leitfähige Source- und Drain-Elektroden an den beiden Enden des Kanals
enthält, wobei der organische Halbleiter wenigstens eine Verbindung der Formel (I) umfasst. Des Weiteren umfasst der organische Feldeffekttransistor in der Regel ein Dielektrikum.

Eine weitere spezielle Ausführungsform ist ein Substrat mit einem Muster von organischen Feldeffekttransistoren, wobei jeder Transistor einen integrierten Schaltkreis bildet oder Teil eines integrierten Schaltkreises ist und wobei zumindest ein Teil der Transistoren wenigstens eine Verbindung der Formel (I) umfasst.

Als Substrate eignen sich prinzipiell die dafür bekannten Materialien. Geeignete Substrate umfassen z. B. Metalle (vorzugsweise Metalle der Gruppen 8, 9, 10 oder 11 des Periodensystems, wie Au, Ag, Cu), oxidische Materialien (wie Glas, Quarz, Keramiken, SiO₂), Halbleiter (z. B. gedoptes Si, gedoptes Ge), Metalllegierungen (z. B. auf Basis von Au, Ag, Cu, etc.), Halbleiterlegierungen, Polymere (z. B. Polyvinylchlorid, Polyolefine, wie Polyethylen und Polypropylen, Polyester, Fluoropolymere, Polyamide, Polyimide, Polyurethane, Polyalkyl(meth)acrylate, Polystyrol und Mischungen und Komposite davon), anorganische Feststoffe (z. B. Ammoniumchlorid), Papier und Kombinationen davon. Die Substrate können flexibel oder unflexibel solid, mit gekrümmter oder planarer Geometrie sein, abhängig von der gewünschten Anwendung.

Ein typisches Substrat für Halbleiterbausteine umfasst eine Matrix (z. B. eine Quarz-oder Polymermatrix) und, optional, eine dielektrische Deckschicht.

Geeignete Dielektrika sind SiO₂, Polystyrol, Poly-α-methylstyrol, Polyolefine (wie Polypropylen, Polyethylen, Polyisobuten), Polyvinylcarbazol, fluorierte Polymere (z. B. Cytop, CYMM) Cyanopullane, Polyvinylphenol, Poly-p-xylol, Polyvinylchlorid oder thermisch oder durch Luftfeuchtigkeit vernetzbare Polymere. Spezielle Dielektrika sind "self assembled nanodielectrics", d. h. Polymere, welche aus SiCl-Funktionalitäten enthaltenden Monomeren wie z. B. Cl₃SiOSiCl₃, Cl₃Si-(CH₂)₆-SiCl₃, Cl₃Si-(CH₂)₁₂-SiCl₃ erhalten und/oder welche durch Luftfeuchtigkeit oder durch Zugabe von Wasser in Verdünnung mit Lösungsmitteln vernetzt werden (siehe z. B. Faccietti Adv. Mat. 2005, 17, 1705-1725). Anstelle von Wasser können auch hydroxylgruppenhaltige Polymere wie Polyvinylphenol oder Polyvinylalkohol oder Copolymere aus Vinylphenol und Styrol als Vernetzungskomponenten dienen. Es kann auch wenigstens ein weiteres Polymer während des Vernetzungsvorgangs zugegen sein, wie z. B. Polystyrol, welches dann mitvernetzt wird (siehe Facietti, US-Patentanmeldung 2006/0202195).

Das Substrat kann zusätzlich Elektroden aufweisen, wie Gate-, Drain- und Source-Elektroden von OFETs, die normalerweise auf dem Substrat lokalisiert sind (z. B. abgeschieden auf oder eingebettet in eine nichtleitende Schicht auf dem Dielektrikum). Das Substrat kann zusätzlich leitfähige Gate-Elektroden der OFETs enthalten, die üblicherweise unterhalb der dielektrischen Deckschicht (d. h. dem Gate-Dielektrikum) angeordnet sind.

Nach einer speziellen Ausführung befindet sich eine Isolatorschicht (gate insulating layer) auf wenigstes einem Teil der Substratoberfläche. Die Isolatorschicht umfasst wenigstens einen Isolator, der vorzugsweise ausgewählt ist unter anorganischen Isolatoren, wie SiO₂, SiN, etc., ferroelektrischen Isolatoren, wie Al₂O₃ Ta₂O₅, La₂O₅ TiO₂, Y₂O₃, etc., organischen Isolatoren, wie Polyimiden, Benzocyclobuten (BCB), Polyvinylalkoholen, Polyacrylaten, etc. und Kombinationen davon.

Geeignete Materialien für Source- und Drain-Elektroden sind prinzipiell elektrisch leitfähige Materialien. Dazu zählen Metalle, vorzugsweise Metalle der Gruppen 8, 9, 10 oder 11 des Periodensystems, wie Pd, Au, Ag, Cu, Al, Ni, Cr, etc. Geeignet sind weiterhin leitfähige Polymere, wie PEDOT (=Poly(3,4-ethylendioxythiophen); PSS (= Poly(styrolsulfonat), Polyanilin, oberflächenmodifiziertes Gold, etc. Bevorzugte elektrisch leitfähige Materialien haben einen spezifischen Widerstand von weniger als 10 ⁻³, vorzugsweise weniger als 10⁻⁴, insbesondere weniger als 10 ⁻⁶ oder 10⁻⁷ Ohm x Meter.

Nach einer speziellen Ausführung befinden sich Drain- und Source-Elektroden zumindest teilweise auf dem organischen Halbleitermaterial. Selbstverständlich kann das Substrat weitere Komponenten umfassen, wie sie üblicherweise in Halbleitermaterialien oder ICs eingesetzt werden, wie Isolatoren, Widerstände, Kondensatoren, Leiterbahnen, etc.

Die Elektroden können nach üblichen Verfahren, wie Verdampfen, lithographische Verfahren oder einen anderen Strukturierungsprozess aufgebracht werden.

Die Halbleitermaterialien können auch mit geeigneten Hilfsmitteln (Polymere, Tenside) in disperser Phase durch Verdrucken verarbeitet werden.

In einer bevorzugten Ausführungsform erfolgt die Abscheidung wenigstens einer Verbindung der allgemeinen Formel (I) (und gegebenenfalls wenigstens einem organischen Halbleitermaterial) durch ein Gasphasenabscheidungsverfahren (Physical Vapor Deposition PVD). PVD-Verfahren werden unter Hochvakuumbedingungen durchgeführt und umfassen die folgenden Schritte: Verdampfen, Transport, Abscheidung. Die Verbindungen der allgemeinen Formel I eignen sich besonders vorteilhaft für einen Einsatz in einem PVD-Verfahren, da sie sich im Wesentlichen nicht zersetzen und/oder unerwünschte Nebenprodukte bilden. Das abgeschiedene Material wird in hoher Reinheit erhalten. Allgemein wird zur PVD wenigstens eine Verbindung der allgemeinen Formel (I), gegebenenfalls in Kombination mit wenigstens einem organischen Halbleiter, auf eine Temperatur oberhalb ihrer Verdampfungstemperatur erhitzt und durch Abkühlen unterhalb der Kristallisationstemperatur auf einem Substrat abgeschieden. Die Temperatur des Substrats bei der Abscheidung liegt vorzugsweise in einem Bereich von etwa 20 bis 250 °C, besonders bevorzugt 50 bis 200 °C.

Die erhaltenen Halbleiterschichten weisen im Allgemeinen eine Dicke auf, die für einen ohmschen Kontakt zwischen Source- und Drain-Elektrode ausreicht. Die Abscheidung kann unter einer Inertatmosphäre, z. B. unter Stickstoff, Argon oder Helium, erfolgen.

Die Abscheidung erfolgt üblicherweise bei Umgebungsdruck oder unter reduziertem Druck. Ein geeigneter Druckbereich beträgt etwa 10⁻⁷ bis 1,5 bar.

Vorzugsweise wird die wenigstens eine Verbindung der Formel (I) enthaltende Schicht auf dem Substrat in einer Dicke von 10 bis 1000 nm, besonders bevorzugt 15 bis 250 nm, abgeschieden.

Die Verbindungen der allgemeinen Formel (I) können auch vorteilhaft aus Lösung prozessiert werden. Das Auftragen wenigstens einer Verbindung der allgemeinen Formel (I) auf ein Substrat (und gegebenenfalls wenigstens eines Halbleitermaterials) erfolgt dann z. B. durch eine Rotationsbeschichtung (spin coating). Die Verbindungen der Formel (I) eignen sich auch zur Herstellung von Halbleiterelementen, speziell OFETs, durch ein Druckverfahren. Es können dafür übliche Druckprozesse (Inkjet, Flexo, Offset, gravure; Tiefdruck, Nanoprint) verwendet werden. Bevorzugte Lösungsmittel für den Einsatz der Verbindungen der Formel (I) in einem Druckverfahren sind aromatische Lösungsmittel wie Toluol, Xylol, etc. Man kann zu diesen "Halbleitertinten" verdickend wirkende Substanzen, wie Polymere zusetzen, z. B. Polystyrol, etc. Dabei verwendet man als Dielektrikum die zuvor genannten Verbindungen.

In einer speziellen Ausführungsform handelt es sich bei dem erfindungsgemäßen Feldeffekttransistor um einen Dünnschichttransistor (thin film transistor, TFT). Gemäß einem üblichen Aufbau verfügt ein Dünnschichttransistor über eine auf dem Substrat befindliche Gate-Elektrode, eine auf dieser und dem Substrat befindliche Gate-Isolierschicht, eine auf der Gate-Isolierschicht befindliche Halbleiterschicht, eine ohmsche Kontaktschicht auf der Halbleiterschicht sowie über eine Source-Elektrode und eine Drain-Elektrode auf der ohmschen Kontaktschicht.

In einer geeigneten Ausführungsform wird die Oberfläche des Substrats vor der Abscheidung wenigstens einer Verbindung der allgemeinen Formel (I) (und gegebenenfalls wenigstens eines organischen Halbleitermaterials) einer Modifizierung unterzogen. Diese Modifizierung dient der Bildung von die Dotierstoffe und/oder Halbleitermaterialien bindenden Bereichen und/oder von Bereichen, auf denen keine Dotierstoffe und/oder Halbleitermaterialien abgeschieden werden können. Bevorzugt wird die Oberfläche des Substrats mit wenigstens einer Verbindung (C1) modifiziert, die geeignet ist, an die Oberfläche des Substrats sowie die Verbindungen der Formel (I) zu binden. In einer geeigneten Ausführungsform wird ein Teil der Oberfläche oder die komplette Oberfläche des Substrats mit wenigstens einer Verbindung (C1) beschichtet, um eine verbesserte Abscheidung wenigstens einer Verbindung der allgemeinen Formel (I) (und gegebenenfalls weiterer halbleitender Verbindungen) zu ermöglichen. Eine weitere Ausführungsform umfasst die Abscheidung eines Musters von Verbindungen der allgemeinen Formel (C1) auf dem Substrat nach einem entsprechenden Herstellungsverfahren. Dazu zählen die dafür bekannten Maskenprozesse sowie so genannte "Pat-terning"-Verfahren, wie sie z. B. in der US-2007-0190783-A1 beschrieben sind, worauf hier in vollem Umfang Bezug genommen wird.

Geeignete Verbindungen der Formel (C1) sind zu einer bindenden Wechselwirkung sowohl mit dem Substrat als auch mit wenigstens einem Dotierstoff der allgemeinen Formel I und/oder wenigstens einer Halbleiterverbindung befähigt. Der Begriff "bindende Wechselwirkung" umfasst die Bildung einer chemischen Bindung (kovalenten Bindung), ionischen Bindung, koordinativen Wechselwirkung, Van der Waals-Wechselwirkungen, z. B. Dipol-Dipol-Wechselwirkungen) etc. und Kombinationen davon. Geeignete Verbindungen der allgemeinen Formel (C1) sind:
- Silane, Phosphonsäuren, Carbonsäuren, Hydroxamsäuren, wie Alkyltrichlorsilane, z. B. n-(Octadecyl)trichlorsilan; Verbindungen mit Trialkoxysilan-Gruppen, z. B. Alkyltrialkoxysilane, wie n-Octadecyltrimethoxysilan, n-Octadecyltriethoxysilan, n-Octadecyltri-(n-propyl)oxysilan, n-Octadecyltri-(isopropyl)oxysilan; Trialkoxyaminoalkylsilane, wie Triethoxyaminopropylsilan und N[(3-triethoxysilyl)-propyl]-ethylendiamin; Trialkoxyalkyl-3-glycidylethersilane, wie Triethoxypropyl-3-glycidylethersilan; Trialkoxyallylsilane wie Allyltrimethoxysilan; Trialkoxy-(isocyanatoalkyl)silane; Trialkoxysilyl(meth)acryloxyalkane und Trialkoxysilyl-(meth)acrylamidoalkane, wie 1-Triethoxysilyl-3-acryloxypropan.
- Amine, Phosphine und Schwefel enthaltende Verbindungen, speziell Thiole.

Bevorzugt ist die Verbindung (C1) ausgewählt unter Alkyltrialkoxysilanen, speziell n-Octadecyltrimethoxysilan, n-Octadecyltriethoxysilan; Hexaalkyldisilazanen, und speziell Hexamethyldisilazan (HMDS); C₈-C₃₀-Alkylthiolen, speziell Hexadecanthiol; Mercaptocarbonsäuren und Mercaptosulfonsäuren speziell Mercaptoessigsäure, 3-Mercaptopropionsäure, Mercaptobernsteinsäure, 3-Mercapto-1-propansulfonsäure und den Alkalimetall- und Ammoniumsalzen davon.

Es sind auch verschiedene Halbleiterarchitekturen mit den erfindungsgemäßen Halbleitern denkbar wie z. B. Top Contact, Top Gate, Bottom Contact, Bottom Gate, oder aber ein vertikaler Aufbau wie z. B. ein VOFET (Vertical organic field effect transistor) wie z. B. in US 2004/0046182 beschrieben.

Die Schichtdicken betragen bei Halbleitern z. B. 10 nm bis 5 µm, beim Dielektrikum 50 nm bis 10 µm, die Elektroden können z. B. 20 nm bis 1 µm dick sein. Die OFETs können auch zu anderen Bauteilen wie Ringoszillatoren oder Inverter kombiniert werden.

Ein weiterer Aspekt der Erfindung ist die Bereitstellung elektronischer Bauteile, die mehrere Halbleiterkomponenten umfassen, wobei es sich um n- und/oder p-Halbleiter handeln kann. Beispiele solcher Bauteile sind Feldeffekttransistoren (FETs), bipolare Flächentransistoren (bipolar junction transistors, BJTs), Tunneldioden, Wechselrichter, lichtemittierende Bauteile, biologische und chemische Detektoren oder Sensoren, temperaturabhängige Detektoren, Photodetektoren wie Polarisations-sensitive Photodetektoren, Gatter, AND-, NAND-, NOT-, OR-, TOR-, und NOR-Gatter, Register, Schalter, Zeitbausteine, statische oder dynamische Speicher und andere dynamische oder sequentielle logische oder andere digitale Bauteile einschließlich programmierbarer Schaltungen.

Ein spezielles Halbleiterelement ist ein Inverter. In der digitalen Logic ist der Inverter ein Gatter, das ein Eingangssignal invertiert. Der Inverter wird auch als NOT-Gate bezeichnet. Reale Inverterschaltungen weisen einen Ausgangsstrom auf, der das Gegenteil zum Eingangsstrom darstellt. Übliche Werte sind z. B. (0, +5V) für TTL-Schaltungen. Die Leistungsfähigkeit eines digitalen Inverters gibt die Spannungstransferkurve (Voltage Transfer Curve VTC) wieder, d. h. der Auftrag von Inputstrom gegen Outputstrom. Idealerweise handelt es sich um eine Stufenfunktion und je näher sich die real gemessene Kurve einer solchen Stufe annähert, desto besser ist der Inverter. In einer speziellen Ausführung der Erfindung werden die Verbindungen der Formel (I) als Dotierstoff für organische Halbleiter in einem Inverter eingesetzt.

Die erfindungsgemäßen Chinonderivate der allgemeinen Formel (I) eignen sich weiterhin besonders vorteilhaft für einen Einsatz in der organischen Photovoltaik (OPV).

In organischen Solarzellen werden durch das Licht nicht direkt freie Ladungsträger erzeugt, sondern es bilden sich zunächst Excitonen, d. h. elektrisch neutrale Anregungszustände in Form von Elektron-Loch-Paaren. Diese Excitonen können an geeigneten Grenzflächen (photoaktiven Grenzflächen) getrennt werden. Zur Excitonentrennung eignen sich prinzipiell organische Donor-Akzeptor-Grenzflächen oder Grenzflächen zu einem anorganischen Halbleiter. Dafür ist es erforderlich, dass Excitonen, die im Volumen des organischen Materials generiert werden, an diese photoaktive Grenzfläche diffundieren können.

Organische Solarzellen sind in der Regel schichtförmig aufgebaut und umfassen in der Regel zumindest die folgenden Schichten: Anode, photoaktive Schicht und Kathode. Diese Schichten befinden sich in der Regel auf einem dafür üblichen Substrat. Die photoaktive Schicht enthält in der Regel wenigstens ein Elektronendonormaterial und wenigstens ein Elektronenakzeptormaterial, die einen Donor-Akzeptor-Übergang ermöglichen. Dieser Übergang kann in Form einer so genannten flat-hetero-junction oder vorzugsweise einer bulk-hetero-junction ausgebildet sein. Organische Solarzellen mit photoaktiven Donor-Akzeptor-Übergängen in Form einer bulk-hetero-junction beruhen darauf, den mittleren Abstand bis zur nächsten Grenzfläche verkleinern. Dazu können Mischschichten aus Donoren und Akzeptoren eingesetzt, die ein interpenetrierendes Netzwerk bilden, in dem interne Donor-Akzeptor-Heteroübergänge möglich sind.

Zusätzlich zu der photoaktiven Schicht kann es eine oder mehrere weitere Schichten geben. Dazu zählen z. B.
- Schichten mit Elektronen leitenden Eigenschaften (ETL, electron transport layer),
- Schichten, die ein löcherleitendes Material (hole transport layer, HTL) enthalten, die nicht absorbieren müssen,
- Excitonen und Löcher blockierende Schichten (z. B. excition blocking layers, EBL), die nicht absorbieren sollen, und
- Multiplikatorschichten (multiplication layers).

Die erfindungsgemäßen organischen Solarzellen enthalten wenigstens ein Chinonderivat der allgemeinen Formel (I). Dieses dient vorzugsweise als Dotierstoff für ein Ladungstransportmaterial in der photoaktiven Schicht und/oder wenigstens einer weiteren Schicht, welche ein organisches Halbleitermaterial enthält. Die erfindungsgemäßen organischen Solarzellen enthalten insbesondere wenigstens ein Chinonderivat der allgemeinen Formel (I) als p-Dotierstoff in der photoaktiven Schicht und/oder einer lochleitenden Schicht.

Geeignete Substrate für organische Solarzellen sind z. B. oxidische Materialien (wie Glas, Keramik, SiO₂, insbesondere Quarz, etc.), Polymere (z. B. Polyvinylchlorid, Polyolefine, wie Polyethylen und Polypropylen, Polyester, Fluorpolymere, Polyamide, Polyurethane, Polyalkyl(meth)acrylate, Polystyrol und Mischungen und Komposite davon) und Kombinationen davon.

Als Elektroden (Kathode, Anode) eignen sich prinzipiell Metalle (vorzugsweise der Gruppen 8, 9, 10 oder 11 des Periodensystems, z. B. Pt, Au, Ag, Cu, Al, In, Mg, Ca), Halbleiter (z. B. dotiertes Si, dotiertes Ge, Indium-Zinn-Oxid (ITO), Gallium-Indium-Zinn-Oxid (GITO), Zink-Indium-Zinn-Oxid (ZITO), etc.), Metalllegierungen (z. B. auf Basis Pt, Au, Ag, Cu, etc., speziell Mg/Ag-Legierungen), Halbleiterlegierungen, etc.

Bevorzugt wird als Material für die dem Licht zugewandte Elektrode (bei normaler Struktur die Anode, bei inverser Struktur die Kathode) ein gegenüber dem einfallendem Licht zumindest teilweise transparentes Material eingesetzt. Dazu zählen speziell Glas und transparente Polymere, wie Polyethylenterephthalat. Die elektrische Kontaktierung erfolgt in der Regel durch Metallschichten und/oder transparente leitfähige Oxide (TCOs). Dazu zählt vorzugsweise ITO, FTO, ZnO, TiO₂, Ag, Au, Pt.

Die dem Licht zugewandte Schicht wird so ausgeführt, dass sie ausreichend dünn ist um nur eine minimale Lichtabsorption zu bewirken, aber dick genug, um einen guten Ladungstransport der extrahierten Ladungsträger zu ermöglichen. Die Dicke der Schicht liegt vorzugsweise in einem Bereich von 20 bis 200 nm.

In einer speziellen Ausführung wird als Material für die dem Licht abgewandte Elektrode (bei normaler Struktur die Kathode, bei inverser Struktur die Anode) ein das einfallende Licht zumindest teilweise reflektierendes Material eingesetzt. Dazu zählen Metallfilme, vorzugsweise aus Ag, Au, Al, Ca, Mg, In und Mischungen davon. Die Dicke der Schicht liegt vorzugsweise in einem Bereich von 50 bis 300 nm.

Geeignete Excitonen und Löcher blockierende Schichten sind z. B. in US 6,451,415 beschrieben. Geeignete Materialien für Excitonenblockerschichten sind z. B. 2,9-Dimethyl-4,7-diphenyl-1,10-phenanthrolin (BCP), 4,7-Diphenyl-1,10-phenanthrolin (Bphen), 1,3-bis[2-(2,2'-bipyridin-6-yl)-1,3,4-oxadiazo-5-yl]benzol (BPY-OXD), Polyethylendioxythiophen (PEDOT), etc. Bevorzugt wird ein Material eingesetzt, das gleichzeitig gut für einen Elektronentransport geeignet ist. Bevorzugt sind BCP, Bphen und BPY-OXD.

Soweit vorhanden, liegt die Dicke der Schichten mit excitonenblockierenden Eigenschaften vorzugsweise in einem Bereich von 1 bis 50 nm, besonders bevorzugt 2 bis 20 nm.

Die erfindungsgemäßen Solarzellen basieren vorzugsweise auf photoaktiven Donor-Akzeptor-Heteroübergängen. Dabei werden HTM (hole transport material, Lochtransportmaterial) und das entsprechende ETM (exciton transport material, Excitonentransportmaterial) so gewählt, dass nach Anregung der Verbindungen ein schneller Elektronenübergang auf das ETM stattfindet. Der Heteroübergang kann flach (glatt) ausgeführt werden (vgl. Two layer organic photovoltaic cell, C. W. Tang, Appl. Phys. Lett., 48 (2), 183-185 (1986) oder N. Karl, A. Bauer, J. Holzäpfel, J. Marktanner, M. Möbus, F. Stölzle, Mol. Cryst. Liq. Cryst., 252, 243-258 (1994).). Der Heteroübergang kann auch als Volumen-Heteroübergang (bulk heterojunction bzw. interpenetrierenes Donor-Akzeptor-Netzwerk, (vgl. z. B. C. J. Brabec, N. S. Sariciftci, J. C. Hummelen, Adv. Funct. Mater., 11 (1), 15 (2001).) ausgeführt werden.

In einer geeigneten Ausführungsform erfolgt die Herstellung der photoaktiven Donor-Akzeptor-Übergänge durch ein Gasphasenabscheidungsverfahren (Physical Vapor Deposition, PVD). Geeignete Verfahren sind z.B. in der US 2005/0227406 beschrieben, worauf hier Bezug genommen wird. Dazu können die Halbleitermaterialien für die photoaktive Schicht einer Gasphasenabscheidung unterzogen werden. Dabei können Halbleitermaterial und Dotierstoff (speziell p-Halbleitermaterial und wenigstens ein Chinonderivat der Formel (I)) auch einer gemeinsamen Gasphasenabscheidung im Sinne einer Co-Sublimation unterzogen werden. Des Weiteren können photoaktive Schichten mit bulk-hetero-junction durch Co-Sublimation wenigstens eines Donormaterials, wenigstens eines Akzeptormaterials und gegebenenfalls wenigstens eines Dotierstoffs hergestellt werden. PVD-Verfahren werden unter Hochvakuumbedingungen durchgeführt und umfassen die folgenden Schritte: Verdampfen, Transport, Abscheidung. Die Abscheidung erfolgt vorzugsweise bei einem Druck im Bereich von etwa 10⁻⁵ bis 10⁻⁷ mbar. Die Abscheidungsrate liegt vorzugsweise in einem Bereich von etwa 0,01 bis 10 nm/s. Die Temperatur des Substrats bei der Abscheidung liegt vorzugsweise in einem Bereich von etwa -100 bis 300 °C, besonders bevorzugt -50 bis 250 °C. Die Abscheidung kann unter einer Inertatmosphäre, z. B. unter Stickstoff, Argon oder Helium, erfolgen.

Die Herstellung der übrigen, die Solarzelle bildenden Schichten kann auch nach üblichen, dem Fachmann bekannten Verfahren erfolgen. Dazu zählt das Aufdampfen im Vakuum oder in Inertgasatmosphäre, Laserablation oder Lösungs- oder Dispersionsprozessierverfahren wie Spin-Coating, Rakeln, Gießverfahren, Aufsprühen, Tauchbeschichtung oder Drucken (z. B. Inkjet, Flexo, Offset, Gravur; Tiefdruck, Nanoimprint).

Die erfindungsgemäßen Solarzellen können als einzelne Zelle mit normaler Struktur vorliegen. In einer speziellen Ausführungsform weist eine solche Zelle den folgenden Schichtaufbau auf:
- ein zumindest teilweise lichtdurchlässiges Substrat,
- eine erste Elektrode (Frontelektrode, Anode)
- eine lochleitende Schicht,
- eine photoaktive Schicht,
- elektronenleitende Schicht,
- eine excitonenblockierende/elektronenleitende Schicht,
- eine zweite Elektrode (Rückelektrode, Kathode).

Die erfindungsgemäßen Sotarzellen können auch als einzelne Zelle mit inverser Struktur vorliegen. In einer speziellen Ausführungsform weist eine solche Zelle den folgenden Schichtaufbau auf:
- ein zumindest teilweise lichtdurchlässiges Substrat,
- eine erste Elektrode (Frontelektrode, Kathode)
- eine excitonenblockierende/elektronenleitende Schicht,
- elektronenleitende Schicht,
- eine photoaktive Schicht,
- eine lochleitende Schicht,
- eine zweite Elektrode (Rückelektrode, Anode).

Die Verbindungen der Formel I können in Solarzellen mit MiM-, pin-, pn-, Mip- oder Min-Aufbau zum Einsatz kommen (M = Metall, p = p-dotierter organischer oder anorganischer Halbleiter, n = n-dotierter organischer oder anorganischer Halbleiter, i = intrinsisch leitfähiges System organischer Schichten, vgl. z. B. J. Drechsel et al., Org. Eletron., 5 (4), 175 (2004) oder Maennig et al., Appl. Phys. A 79, 1-14 (2004)).

Die Verbindungen der Formel I können auch in Tandemzellen, wie von P. Peumans, A. Yakimov, S. R. Forrest in J. Appl. Phys, 93 (7), 3693-3723 (2003) (vgl. Patente US 4,461,922, US 6,198,091 und US 6,198,092) beschrieben, verwendet werden.

Die Verbindungen der Formel I können auch in Tandemzellen aus zwei oder mehreren aufeinandergestapelten MiM-, pin-, Mip- oder Min-Dioden (vgl. Patentanmeldung DE 103 13 232.5) verwendet werden (J. Drechsel et al., Thin Solid Films, 451452, 515-517 (2004)).

Die Schichtdicken der M, n, i und p-Schichten betragen typischerweise 10 bis 1000 nm, bevorzugt 10 bis 400 nm, besonders bevorzugt 10 bis 100 nm.

Geeignete Halbleitermaterialien für die organischen Solarzellen sind die zuvor genannten, worauf hier Bezug genommen wird.

Die Efindung wird anhand der folgenden nicht einschränkenden Beispiele näher erläutert.

### Beispiele

### I) Herstellung von Chinonderivaten

### Beispiel 1:

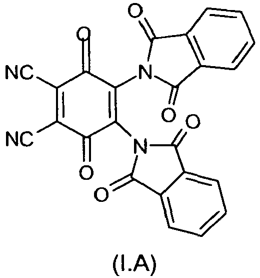

33,4 g (147 mMol) 2,3-Dichlor-5,6-dicyano-benzoquinon (DDQ) wurden gelöst in 500 ml Acetonitril vorgelegt und mit 54,5 g (294 mMol) Phthalimid-Kaliumsalz versetzt. Es wurde zum Rückfluss erhitzt und 12 Stunden gerührt, wobei eine dunkelbraune Suspension erhalten wurde. Nach dem Abkühlen wurde der Niederschlag abgesaugt, der Rückstand in 500 ml Wasser suspendiert und 1 Stunde bei 90 °C gerührt. Es wurde heiß abgesaugt und mit ca. 1000 ml heißem Wasser in mehreren Portionen nachgewaschen. Anschließend wurde einmal mit wenig Ethanol gewaschen und das Produkt im Vakuumtrockenschrank bei 100 °C getrocknet. Man erhielt 9,3 g eines gelben Feststoffs.
Thermostabilität: > 400 °C
Sublimationstemperatur: 240 °C

### II) Anwendungstechnische Eigenschaften

### Beispiel 2:

Diffusions(Migrations)messungen an der Verbindung (I.A) und an F₆TNAP als Vergleich mit UPS

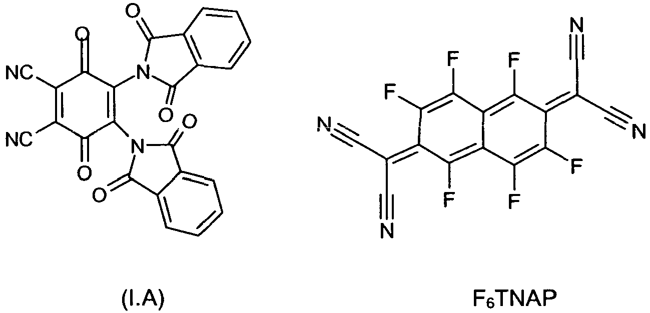

Für die Diffusionsmessungen wurden die folgenden Proben eingesetzt. Als Substrat diente jeweils ein mit einer 50 nm Silberschicht beschichteter Si-Wafer.

### Vergleich:

1) 10 nm reines NPD (N,N'-Bis-1-(naphthyl)-N,N'-diphenyl-1,1'-biphenyl-4,4'-diamin)
2) 10 nm NPD, dotiert mit 2 Gew.-% F₆TNAP
3) 30 nm reines NPD aufgedampft auf eine Schicht aus 10 nm NPD, dotiert mit 2 Gew.-% F₆TNAP

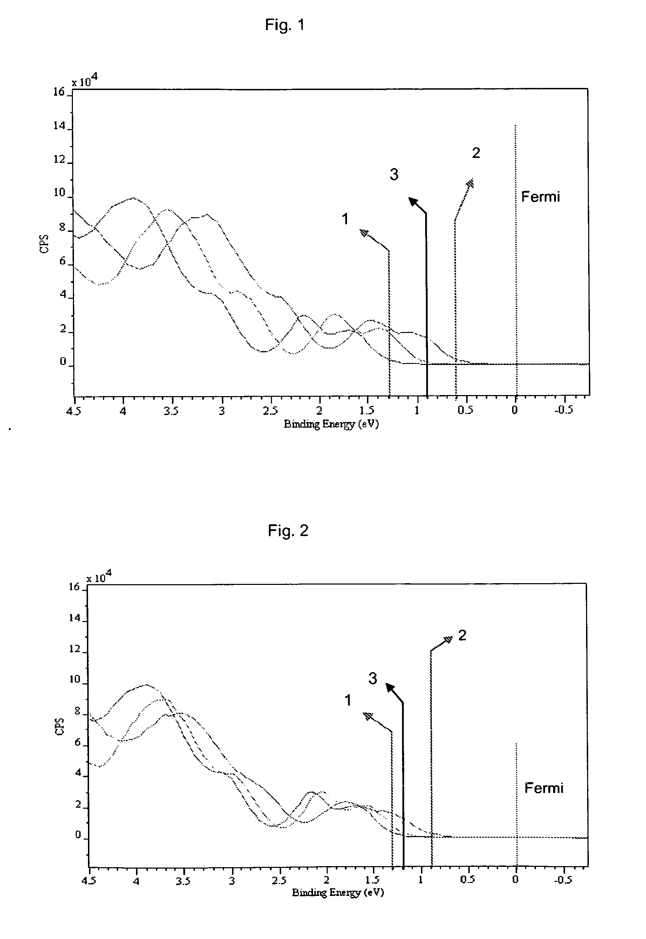

Die Ergebnisse sind in Figur 1 wiedergegeben.

Ob der Dotierstoff in die benachbarte undotierte Schicht wandert, kann direkt mittels UPS bestimmt werden. Aus XPS Messungen ist bekannt, dass der Vergleichsdotierstoff F₆TNAP zur Diffusion neigt. Dies konnte durch die UPS-Messungen an den drei oben genannten Substraten bestätigt werden.

Das HOMO der reinen 10 nm NPD Schicht (1) hat eine Energiedifferenz von 1,3 eV zum Ferminiveau. Das HOMO des Substrats (2) (10 nm NPD, dotiert mit 2 Gew.-% F₆TNAP) hat aufgrund der Dotierung nur eine Energiedifferenz von 0,6 eV. Ohne eine Migration von Dotierstoff in die obere NPD-Schicht sollte das Substrat (3) dasselbe HOMO wie Substrat (1) aufweisen. Die gemessene Energiedifferenz zwischen HOMO und Ferminiveau beträgt jedoch 0,9 eV und ist somit 0,4 eV kleiner als die Differenz zwischen Ferminiveau und reinem NPD. Dies belegt eine signifikante Diffusion von F₆TNAP in die 30 nm NPD-Schicht.

### Erfindungsgemäß:

1) 10 nm reines NPD
2) 10 nm NPD, dotiert mit 2 Gew.-% Chinonderivat (I.A)
3) 30 nm reines NPD aufgedampft auf eine Schicht aus 10 nm NPD, dotiert mit 2 Gew.-% Chinonderivat (I.A)

Die Ergebnisse sind in Figur 2 wiedergegeben.

Die gemessene Energiedifferenz zwischen HOMO des Substrats (3) und Ferminiveau ist nur um 0,1 eV kleiner als die Differenz zwischen Ferminiveau und reinem NPD. Dies belegt, dass das erfindungsgemäße Chinonderivat (1A) keine signifikante Diffusion in die 30 nm NPD-Schicht aufweist.

**Tabelle 1: Vergleich mit kommerziellen Dotierstoffen**

| Dotierstoff | F4-TCNQ | MoO₃ | (I.A) |
|---|---|---|---|
| Mg. | 276,15 | - | 448,35 |
| IP | 7,82 eV | - | 7,45 eV |
| EA | 4,96 eV | - | 4,82 eV |
| Verdampfungstemperatur im Hochvakuum (10⁻⁷mbar) | weniger als 80 °C (leicht kontaminierbar) | 470 ±10 °C | 190 ±10 °C |

| | | | |
|---|---|---|---|
| Mg. = Molekulargewicht EA = Elektronenaffinität IP = Ionisierungspotential | | | |

F4-TCNQ hat eine zu geringe, MoO₃ eine zu hohe Sublimationstemperatur. Beide eignen sich nur eingeschränkt für einen Einsatz in OLEDs.

## Patentansprüche

1. Chinonderivat der allgemeinen Formel (I) 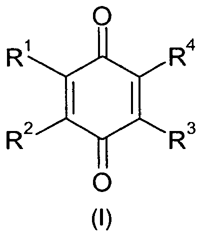 worin
1, 2 oder 3 der Reste R¹ bis R⁴ für CN stehen,
1, 2 oder 3 der Reste R¹ bis R⁴ unabhängig voneinander für einen Phthalimidrest der allgemeinen Formel (II) 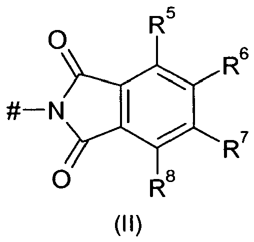 stehen, worin
# für die Verknüpfungsstelle zu einem Ringkohlenstoffatom des Chinonrings steht,
R⁵, R⁶, R⁷ und R⁸ unabhängig voneinander für Wasserstoff, Fluor, Chlor, Brom, Cyano, Nitro, Nitroso, Formyl, Acyl, COOH, Carboxylat, Alkylcarbonyloxy, Carbamoyl, SO₃H Sulfonat, Sulfamino, Sulfamid, Amidino, NE¹E², oder für unsubstituiertes oder substituiertes Alkyl, Alkoxy, Alkylamino, Alkylthio, Alkylsulfinyl, Alkylsulfonyl, Alkenyl, Alkadienyl, Alkinyl, Cycloalkyl, Bicycloalkyl, Cycloalkenyl, Heterocycloalkyl, Aryl oder Heteroaryl stehen, wobei E¹ und E² unabhängig voneinander für Wasserstoff, Alkyl, Cycloalkyl oder Aryl stehen, wobei jeweils zwei benachbarte Reste R⁵ bis R⁸ gemeinsam mit den Kohlenstoffatomen des Benzolkerns, an die sie gebunden sind, auch für ein kondensiertes Ringsystem mit 1, 2 oder 3 weiteren Ringen stehen können, und
die Reste R¹ bis R⁴, soweit vorhanden, die nicht für CN oder für einen Phthalimidrest der allgemeinen Formel (II) stehen, unabhängig voneinander ausgewählt sind unter Wasserstoff, Fluor, Chlor, Brom, Cyano, Nitro, Nitroso, Formyl, Acyl, COOH, Carboxylat, Alkylcarbonyloxy, Carbamoyl, SO₃H, Sulfonat, Sulfamino, Sulfamid, Amidino, NE³E⁴, oder unsubstituiertem oder substituiertem Alkyl, Alkoxy, Alkylamino, Alkylthio, Alkylsulfinyl, Alkylsulfonyl, Alkenyl, Alkadienyl, Alkinyl, Cycloalkyl, Bicycloalkyl, Cycloalkenyl, Heterocycloalkyl, Aryl oder Heteroaryl, wobei E³ und E⁴ unabhängig voneinander für Wasserstoff, Alkyl, Cycloalkyl oder Aryl stehen,
wobei R¹ und R² und/oder R³ und R⁴ gemeinsam mit den Kohlenstoffatomen des Chinonrings, an die sie gebunden sind, auch für ein kondensiertes Ringsystem mit 1, 2 oder 3 weiteren Ringen stehen können.

2. Chinonderivat nach Anspruch 1, wobei keiner der Reste R¹ bis R⁴ für Halogen steht.

3. Chinonderivat nach einem der vorhergehenden Ansprüche, wobei zwei der Reste R¹ bis R⁴ für Cyano stehen.

4. Chinonderivat nach einem der vorhergehenden Ansprüche, wobei zwei der Reste R¹ bis R⁴ für einen Phthalimidrest der allgemeinen Formel (II) stehen.

5. Chinonderivat nach einem der vorhergehenden Ansprüche, wobei keiner der Reste R⁵ bis R⁸ für Halogen steht.

6. Chinonderivat der allgemeinen Formel (I.A)

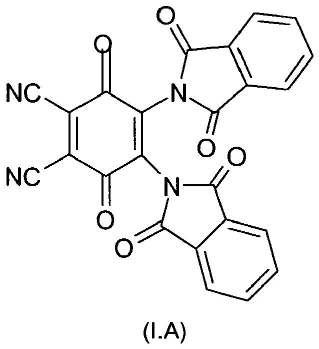

7. Verfahren zur Herstellung von Chinonderivaten der allgemeinen Formel (I) 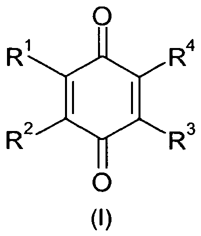 wobei R¹ bis R⁴ wie in einem der Ansprüche 1 bis 6 definiert sind,
bei dem man eine Verbindung der Formel (I), bei der wenigstens einer der Reste R¹ bis R⁴ für Cl oder Br steht, einer Umsetzung mit einer Phthalimidverbindung der allgemeinen Formel (II.a) 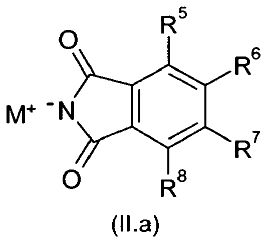 unterzieht, worin
M⁺ für ein Kationäquivalent steht,
R⁵, R⁶, R⁷ und R⁸ wie in Anspruch 1 oder 5 definiert sind.

8. Verwendung von Chinonderivaten der allgemeinen Formel (I), wie in einem der Ansprüche 1 bis 6 definiert, oder erhalten nach einem Verfahren, wie in Anspruch 7 definiert, als Dotierstoff in der organischen Elektronik.

9. Verwendung nach Anspruch 8 als Dotierstoff für organische halbleitende Materialien, vorzugsweise als p-Dotierstoff für ein Lochleitermaterial.

10. Organische Leuchtdiode enthaltend mindestens ein Chinonderivat der allgemeinen Formel (I), wie in einem der Ansprüche 1 bis 6 definiert, oder erhalten nach einem Verfahren, wie in Anspruch 7 definiert.

11. Lochleitende Schicht enthaltend mindestens ein Chinonderivat der allgemeinen Formel (I), wie in einem der Ansprüche 1 bis 6 definiert, oder erhalten nach einem Verfahren, wie in Anspruch 7 definiert.

12. Vorrichtung, ausgewählt aus der Gruppe bestehend aus stationären Bildschirmen, wie Bildschirmen von Computern, Fernsehern, Bildschirmen in Druckern, Küchengeräten sowie Reklametafeln; Beleuchtungen; Hinweistafeln und mobilen Bildschirmen, wie Bildschirmen in Handys, Laptops, Digitalkameras, Fahrzeugen sowie Zielanzeigen an Bussen und Bahnen, enthaltend mindestens eine organische Leuchtdiode gemäß Anspruch 10 oder eine lochleitende Schicht gemäß Anspruch 11.

13. Verwendung von wenigstens einem Chinonderivat der allgemeinen Formel (I), wie in einem der Ansprüche 1 bis 6 definiert, oder erhalten nach einem Verfahren, wie in Anspruch 7 definiert, in organischen Leuchtdioden, bevorzugt in einer Leiterschicht, besonders bevorzugt einer Lochleiterschicht.

14. Verwendung von wenigstens einem Chinonderivat der allgemeinen Formel (I), wie in einem der Ansprüche 1 bis 6 definiert, oder erhalten nach einem Verfahren, wie in Anspruch 7 definiert, in organischen Feldeffekttransistoren.

15. Verwendung von wenigstens einem Chinonderivat der allgemeinen Formel (I), wie in einem der Ansprüche 1 bis 6 definiert, oder erhalten nach einem Verfahren, wie in Anspruch 7 definiert, als Dotierstoff in der organischen Photovoltaik, insbesondere als Dotierstoff für ein Ladungstransportmaterial.

16. Organischer Feldeffekttransistor, umfassend ein Substrat mit wenigstens einer Gate-Struktur, einer Source-Elektrode und einer Drain-Elektrode und wenigstens einer Verbindung der Formel (I), wie in einem der Ansprüche 1 bis 6 definiert, oder erhalten nach einem Verfahren, wie in Anspruch 7 definiert, als Dotierstoff.

17. Substrat mit einer Vielzahl von organischen Feldeffekttransistoren, wobei zumindest ein Teil der Feldeffekttransistoren wenigstens eine Verbindung der Formel (I), wie in einem der Ansprüche 1 bis 6 definiert, oder erhalten nach einem Verfahren, wie in Anspruch 7 definiert, als Dotierstoff enthält.

18. Halbleiterbaustein, umfassend wenigstens ein Substrat, wie in Anspruch 17 definiert.

19. Organische Solarzelle, enthaltend wenigstens einer Verbindung der Formel (I), wie in einem der Ansprüche 1 bis 6 definiert, oder erhalten nach einem Verfahren, wie in Anspruch 7 definiert.

## Claims

1. A quinone derivative of general formula (I) 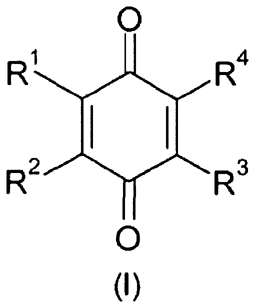 wherein
1, 2 or 3 of the radicals R¹ to R⁴ stand for CN,
1, 2, or 3 of the radicals R¹ to R⁴, independently of one another, stand for a phthalimide
radical of general formula (II) 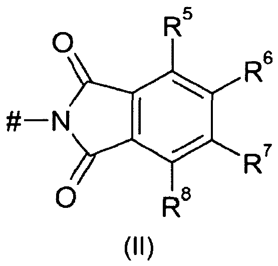 wherein
# stands for the linking point to a ring carbon atom of the quinone ring,
R⁵, R⁶, R⁷ and R⁸, independently of one another, stand for hydrogen, fluorine, chlorine, bromine, cyano, nitro, nitroso, formyl, acyl, COOH, carboxylate, alkylcarbonyloxy, carbamoyl, SO₃H, sulphonate, sulphamino, sulphamide, amidino, NE¹E², or for unsubstituted or substituted alkyl, alkoxy, alkylamino, alkylthio, alkylsulphinyl, alkylksulphonyl, alkenyl, alkadienyl, alkynyl, cycloalkyl, bicycloalkyl, cycloalkenyl, heterocycloalkyl, aryl or heteroaryl, wherein E¹ and E², independently of one another, stand for hydrogen, alkyl, cycloalkyl or aryl,
wherein in each case two adjacent radicals R⁵ to R⁸, together with the carbon atom of the benzene nucleus to which they are bound, may also stand for a condensed ring system containing 1, 2 or 3 further rings, and
the radicals R¹ to R⁴, where provided, which do not stand for CN or for a phthalimide radical of general formula (II), are selected independently of one another from hydrogen, fluorine, chlorine, bromine, cyano, nitro, nitroso, formyl, acyl, COOH, carboxylate, alkylcarbonyloxy, carbamoyl, SO₃H, sulphonate, sulphamino, sulphamide, amidino, NE³E⁴, or unsubstituted or substituted alkyl, alkoxy, alkylamino, alkylthio, alkylsulphinyl, alkylksulphonyl, alkenyl, alkadienyl, alkynyl, cycloalkyl, bicycloalkyl, cycloalkenyl, heterocycloalkyl, aryl or heteroaryl, wherein E³ and E⁴, independently of one another, stand for hydrogen, alkyl, cycloalkyl or aryl,
wherein R¹ and R² and/or R³ and R⁴, together with the carbon atom of the quinone ring to which they are bound, may also stand for a condensed ring system containing 1, 2 or 3 further rings.

2. The quinone derivative according to claim 1, wherein none of the radicals R¹ to R⁴ stands for halogen.

3. The quinone derivative according to any one of the preceding claims, wherein two of the radicals R¹ to R⁴ stand for cyano.

4. The quinone derivative according to any one of the preceding claims, wherein two of the radicals R¹ to R⁴ stand for a phthalimide radical of general formula (II).

5. The quinone derivative according to any one of the preceding claims, wherein none of the radicals R⁵ to R⁸ stands for halogen.

6. The quinone derivative of general formula (I.A)

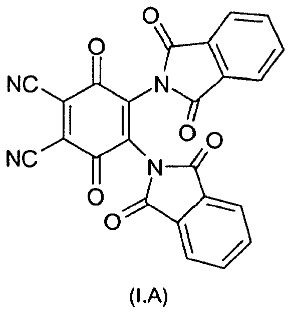

7. A method for producing quinone derivatives of general formula (I) 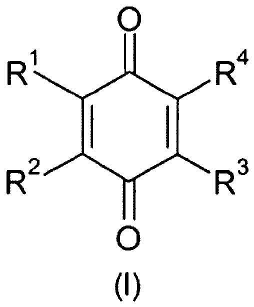 wherein R¹ to R⁴ are as defined in any one of claims 1 to 6,
in which a compound of formula (I), in which at least one of the radicals R¹ to R⁴ stands for Cl or Br, is reacted with a phthalimide compound of general formula (II.a) 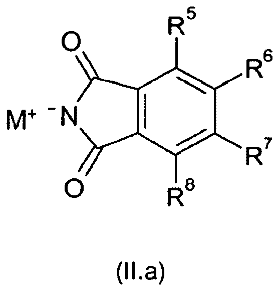 wherein
M⁺ stands for a cation equivalent, and
R⁵, R⁶, R⁷ and R⁸ are as defined in claims 1 or 5.

8. A use of quinone derivatives of general formula (I), as defined in any one of claims 1 to 6, or obtained by a method as defined in claim 7 as a dopant in organic electronics.

9. The use according to claim 8 as a dopant for organic semiconductive materials, preferably as a p-dopant for a hole conductor material.

10. An organic light-emitting diode containing at least one quinone derivative of general formula (I), as defined in any one of claims 1 to 6 or obtained by a method as defined in claim 7.

11. A hole-conducting layer containing at least one quinone derivative of general formula (I), as defined in any one of claims 1 to 6 or obtained by a method as defined in claim 7.

12. A device, selected from the group consisting of stationary visual display units such as visual display units of computers, televisions, visual display units in printers, kitchen appliances and advertising panels; illuminations; information panels and mobile visual display units such as visual display units in mobile telephones, laptops, digital cameras, vehicles and destination displays on buses and trains, comprising at least one organic light-emitting diode according to claim 10 or a hole-conducting layer according to claim 11.

13. A use of at least one quinone derivative of general formula (I), as defined in any one of claims 1 to 6 or obtained by a method as defined in claim 7, in organic light-emitting diodes, preferably in a conductor layer, more preferably a hole conductor layer.

14. A use of at least one quinone derivative of general formula (I), as defined in any one of claims 1 to 6 or obtained by a method as defined in claim 7, in organic field-effect transistors.

15. A use of at least one quinone derivative of general formula (I), as defined in any one of claims 1 to 6 or obtained by a method as defined in claim 7, as a dopant in organic photovoltaics, in particular as a dopant for a charge transport material.

16. An organic field-effect transistor, comprising a substrate having at least a gate structure, a source electrode and a drain electrode and at least one compound of formula I, as defined in any one of claims 1 to 6 or obtained by a method as defined in claim 7, as a dopant.

17. A substrate having a large number of organic field-effect transistors, wherein at least some of the field-effect transistors contain at least one compound of formula (I), as defined in any one of claims 1 to 6 or obtained by a method as defined in claim 7, as a dopant.

18. A semiconductor component, comprising at least one substrate as defined in claim 17.

19. An organic solar cell, containing at least one compound of formula (I) as defined in any one of claims 1 to 6 or obtained by a method as defined in claim 7.

## Revendications

1. Dérivé de quinone répondant à la formule générale (I) 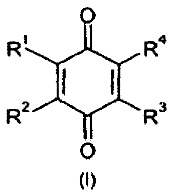 dans laquelle
1, 2 ou 3 des radicaux R¹ à R⁴ représentent CN,
1, 2 ou 3 des radicaux R¹ à R⁴ représentent, indépendamment l'un de l'autre, un radical de phtalimide répondant à la formule générale (II) 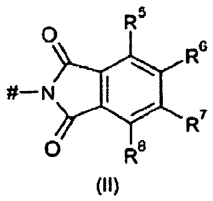 dans laquelle
# Représente un site de liaison avec un atome de carbone faisant partie du cycle de quinone,
R⁵, R⁶, R⁷ et R⁸ représentent, indépendamment l'un de l'autre, un hydrogène, fluor, chlore, brome, cyano, nitro, nitroso, formyle, acyle, COOH, carboxylate, alkylcarbonyloxy, carbamoyle, SO₃H, sulfonate, sulfamino, sulfamide, amidino, NE¹E² ou un alkyle, alkoxy, alkylamino, alkylthio, alkylsulfinyle, alkylsulfonyle, alcényle, alcadiényle, alcynyle, cycloalkyle, bicycloalkyle, cycloalcényle, hétérocycloalkyle, aryle ou hétéroaryle substitué ou non-substitué, E¹ et E² représentant indépendamment l'un de l'autre un hydrogène, alkyle, cyanoalkyle ou aryle,
deux quelconques radicaux voisins parmi R⁵ à R⁸ pouvant également former, ensemble avec les atomes de carbone du noyau de benzène auxquels ils sont liés, un système de cycles condensé comportant 1, 2 ou 3 autres cycles, et
les radicaux R¹ à R⁴ ne représentant pas CN ni un radical de phtalimide répondant à la formule générale (II), étant le cas échéant choisis parmi un hydrogène, fluor, chlore, brome, cyano, nitro, nitroso, formyle, acyle, COOH, carboxylate, alkylcarbonyloxy, carbamoyle, SO₃H, sulfonate, sulfamino, sulfamide, amidino, NE³E⁴ ou un alkyle, alkoxy, alkylamino, alkylthio, alkylsulfinyle, alkylsulfonyle, alcényle, alcadiényle, alcynyle, cycloalkyle, bicycloalkyle, cycloalcényle, hétérocycloalkyle, aryle ou hétéroaryle substitué ou non-substitué, E³ et E⁴ représentant indépendamment l'un de l'autre un hydrogène, alkyle, cycloalkyle ou aryle,
R¹ et R² et/ou R³ et R⁴ pouvant également représenter, ensemble avec les atomes de carbone du cycle de quinone auxquels ils sont liés, un système de cycles condensé comportant 1, 2 ou 3 autres cycles.

2. Dérivé de quinone selon la revendication 1, aucun des radicaux R¹ à R⁴ ne représentant un halogène.

3. Dérivé de quinone selon l'une des revendications précédentes, deux des radicaux R¹ à R⁴ représentant un cyano.

4. Dérivé de quinone selon l'une des revendications précédentes, deux des radicaux R¹ à R⁴ représentant un radical de phtalimide répondant à la formule générale (II).

5. Dérivé de quinone selon l'une des revendications précédentes, aucun des radicaux R⁵ à R⁸ ne représentant un halogène.

6. Dérivé de quinone répondant à la formule générale (I.A)

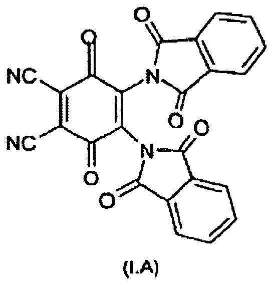

7. procédé de préparation de dérivés de quinone répondant à la formule générale (I) 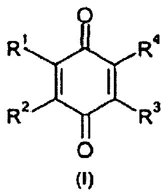 R¹ à R⁴ correspondant aux définitions dans l'une des revendications 1 à 6,
consistant à soumettre un composé de formule (I), dans laquelle au moins un des radicaux R¹ à R⁴ représente Cl ou Br, à une réaction avec un compose de phtalimide répondant à la formule générale (II.a) 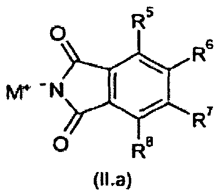 dans laquelle
M⁺ représente un équivalent de cation,
R⁵, R⁶, R⁷ et R⁸ correspondant aux définitions dans les revendications 1 ou 5.

8. Utilisation de dérivés de quinone répondant à la formule générale (I), tels qu'ils sont définis dans l'une des revendications 1 à 6 ou ont été obtenus selon un procédé correspondant à la définition dans la revendication 7, en tant que dopants dans l'électronique organique.

9. Utilisation selon la revendication 8 en tant que dopant destiné à des matériaux organiques semi-conducteurs, préférentiellement en tant que dopant de type p destiné à un matériau conducteur à trous d'électrons.

10. Diode électroluminescente organique, contenant au moins un dérivé de quinone répondant à la formule générale (I), tel qu'il est défini dans l'une des revendications 1 à 6 ou a été obtenu selon un procédé correspondant à la définition dans la revendication 7.

11. Couche conductrice à trous d'électrons, contenant au moins un dérivé de quinone répondant à la formule générale (I), tel qu'il est défini dans l'une des revendications 1 à 6 ou a été obtenu selon un procédé correspondant à la définition dans la revendication 7.

12. Dispositif choisi dans le groupe constitué d'écrans fixes tels que les écrans pour ordinateurs, téléviseurs, les écrans intégrés à des imprimantes, à des appareils de cuisine ainsi qu'à des panneaux publicitaires ; de dispositifs d'éclairage ; de panneaux indicateurs et d'écrans mobiles tels que les écrans intégrés à des téléphones mobiles, à des appareils photo numériques, à des véhicules ainsi qu'à des dispositifs permettant d'afficher la destination de bus et de trains, contenant au moins une diode électroluminescente selon la revendication 10 ou une couche conductrice à trous d'électrons selon la revendication 11.

13. Utilisation d'au moins un dérivé de quinone répondant à la formule générale (I), tel qu'il est défini dans l'une des revendications 1 à 6 ou a été obtenu selon un procédé correspondant à la définition dans la revendication 7, de préférence dans une couche conductrice, avec une préférence particulière dans une couche conductrice à trous d'électrons.

14. Utilisation d'au moins un dérivé de quinone répondant à la formule générale (I), tel qu'il est défini dans l'une des revendications 1 à 6 ou a été obtenu selon un procédé correspondant à la définition dans la revendication 7, dans des transistors à effet de champ de type organique.

15. Utilisation d'au moins un dérivé de quinone répondant à la formule générale (I), tel qu'il est défini dans l'une des revendications 1 à 6 ou a été obtenu selon un procédé correspondant à la définition dans la revendication 7, notamment en tant que dopant destiné à un matériau de transport de charges.

16. Transistor à effet de champ de type organique, comprenant un substrat pourvu d'une structure de grille, d'une électrode de source et d'une électrode de drain et d'au moins un composé de formule (I) tel qu'il est défini dans l'une des revendications 1 à 6 ou a été obtenu selon un procédé correspondant à la définition dans la revendication 7.

17. Substrat comportant une pluralité de transistors à effet de champ de type organique, au moins une partie desdits transistors à effet de champ contenant au moins un composé de formule (I), tel qu'il est défini dans l'une des revendications 1 à 6 ou a été obtenu selon un procédé correspondant à la définition dans la revendication 7, en tant que dopant.

18. Composant semi-conducteur, comprenant au moins un substrat tel qu'il est défini dans la revendication 17.

19. Cellule solaire organique, contenant au moins un composé de formule (I), tel qu'il est défini dans l'une des revendications 1 à 6 ou a été obtenu selon un procédé correspondant à la définition dans la revendication 7.
